# EUROPEAN PATENT APPLICATION

(11) **EP 4 796 143 A2**
(43) Date of publication of application: **26.08.2026**
(21) Application number: 26191913.8
(22) Date of filing: 20.10.2021
(51) Int. Cl.: A61M 5/142

(54) **INSULIN PUMP FOR ENSURED SITE HEALTH AND BLOOD GLUCOSE CONTROL FOR AID SYSTEMS**

(30) Priority: 22.10.2020 US 202063104078 P
(62) Document number(s) of the earlier application(s) in accordance with Art. 76 EPC: 21810478.4 / 4 232 119
(71) Applicant: Insulet Corporation, Acton, MA 01720 (US)
(72) Inventor: NARAYANASWAMI, Rangarajan, Weston, 02493 (US)
(74) Representative: Peterreins Schley

(57) **Abstract**

Disclosed are systems, devices, methods and computer-readable medium products that implement a vibrational event by a wearable drug delivery device. The wearable drug delivery device includes vibrational actuators that vibrate in response to a control signal from a controller. The controller may be controlled by an external device that issues command signals with instructions related to settings of the vibrational event. The vibrational event settings may be modified based on signals received from sensors on the wearable drug delivery device. The vibrational events may be implemented as an element of a site maintenance plan that alleviates the degeneration of tissue at one or more body attachment sites of the wearable drug delivery device.

## Description

### CROSS-REFERENCE TO RELATED APPLICATIONS

This application claims the benefit to U.S. Provisional Application No. 63/104,078, filed October 22, 2020, the entire contents of which are incorporated herein by reference in their entirety.

### TECHNICAL FIELD

The described examples provide a device and techniques for an automatic insulin delivery (AID) system to mitigate degradation and atrophy at the site of placement of a wearable drug delivery device.

### BACKGROUND

A wearable drug delivery device (insulin pump) is mounted with adhesive on a site of the user's body for usually up to three days with wearable drug delivery device sites on the body including the abdomen, the lower back, the upper arms, and thighs. Some users experience irritation and soreness at the site where the wearable drug delivery device is located, and this irritation may gradually lead to site degradation and atrophy that may render the particular site unsuitable for future placement of the wearable drug delivery device.

In addition, site degradation related to frequent placement of the wearable drug delivery device at a particular site increases the chances of occurrence of insulin infusion occlusion. Some wearable drug delivery systems as well as some syringe-based systems have attempted to use vibrations to disrupt an occlusion. In these systems, a vibration generator is attached to a cannula or needle that is intended to disrupt tissue at the tip of the cannula or needle in an effort to move the tip of the cannula or needle away from tissue that may occlude the cannula or needle or to create small fractures in the tissue near the tip of the cannula or needle, which may enable a drug to be administered with lower pressure.

It would be beneficial to have a wearable drug delivery device that is configured to help alleviate skin irritations, rashes or the like as well as mitigate the possibility of insulin infusion occlusion.

### SUMMARY

Disclosed is a wearable drug delivery device including a controller, a memory, a vibrational actuator, a reservoir, and at least one housing. The at least one housing is configured to contain the controller, the memory, the vibrational actuator, and the reservoir. The at least one housing may have a bottom surface to which is disposed an adhesive layer configured to affix to skin of a user, the vibrational actuator may be coupled to the controller and configured to generate vibrations in response to a control signal from the controller. The memory is coupled to the controller and configured to store programming code and a site maintenance application. The reservoir may be configured to store a therapeutic drug. The controller when executing the site maintenance application may be configured to control the vibrational actuator to generate vibrations that extend below the bottom surface of the at least one housing into the skin of the user. The generated vibrations may have a duration and a frequency.

Disclosed is an example of a non-transitory computer readable medium that is embodied with programming code executable by a processor. The processor may be operable to establish a wireless connection with a controller of a wearable drug delivery device. Settings may be determined to implement a vibrational event for the wearable drug delivery device. The determined settings may include a duration of the vibrational event and a frequency of a vibration to be generated during the vibrational event. A command signal containing instructions for the controller of a wearable drug delivery device may be output to actuate vibrational actuators to implement the vibrational event according to the determined settings within the wearable drug delivery device.

Disclosed is a system that includes a personal diabetes management device and a wearable drug delivery device. The personal diabetes management device may include a processor; a memory, a touchscreen display device, and a transceiver. The memory may store programming code and a site maintenance application. The programming code and the site maintenance application may be executable by the processor. The touchscreen display device may be coupled to the processor and configured to receive inputs and present a graphical user interface. The transceiver may be coupled to the processor and be configured to receive and transmit signals containing information of the site maintenance application. The wearable drug delivery device may include a drug delivery device transceiver, a reservoir, a drive mechanism, a vibrational actuator and a controller. The drug delivery device transceiver may be configured to be coupled via a wireless communication link with the personal diabetes management device. The reservoir may be configured to contain insulin. The drive mechanism may be coupled to the reservoir and configured to expel the insulin from the reservoir. The vibrational actuator may be configured to generate vibrations. The controller may be coupled to the drug delivery device transceiver, the drive mechanism and the vibrational actuator. The controller may be configured to receive command signals from the personal diabetes management device. The processor of the personal diabetes management device when executing programming code may be configured to, prior to transmitting a command signal causing the drive mechanism to deliver a bolus dosage of insulin from the reservoir, transmit according to a vibrational event schedule a vibrational event command signal to the wearable drug delivery device. The vibrational event command signal may include a vibration duration and a vibration frequency. The controller of the wearable drug delivery device may be configured to, in response to the vibrational event command signal, send a control signal to actuate the vibrational actuator.

### BRIEF DESCRIPTION OF THE DRAWINGS

FIG. 1A shows an example of a wearable drug delivery device equipped with an example of a first type of vibrational actuator.
FIG. 1B shows another example of a wearable drug delivery device equipped with multiple vibrational actuators of the first type shown in FIG. 1A.
FIG. 1C shows an example of a wearable drug delivery device equipped with an example of a second type of vibrational actuator.
FIG. 1D shows another example of a wearable drug delivery device equipped with multiple vibrational actuators of the second type shown in FIG. 1C.
FIG. 1E shows a further example of a wearable drug delivery device equipped with a combination of first type and second type vibrational actuators such as those shown in the examples of FIGs. 1A and 1C.
FIG. 2A illustrates a cross-sectional, functional block diagram of an example of a wearable drug delivery device that is suitable for implementing the example processes and techniques described herein.
FIG. 2B illustrates a cross-sectional, functional block diagram of another example of a wearable drug delivery device that is suitable for implementing example processes and techniques described herein.
FIG. 2C illustrates a cross-sectional, functional block diagram of an example of a wearable analyte sensor that is suitable for implementing the example vibrational processes and techniques described herein.
FIG. 3A shows a flow chart of an example process for actuating a vibrational actuator within a wearable drug delivery device.
FIG. 3B illustrates a flow chart of another example process for actuating a vibrational actuator within a wearable drug delivery device.
FIG. 4A illustrates a flow chart of another example process for establishing a vibrational event schedule.
FIG. 4B illustrates an example of a graphical user interface presented on an example management device as described herein.
FIG. 4C illustrates a flow chart of yet another example process for establishing a vibrational event schedule.
FIG. 5 illustrates a functional block diagram of a system example suitable for implementing the example processes and techniques described herein.

### DETAILED DESCRIPTION

Various examples provide methods, a system, devices and a computer-readable medium for actuating vibrational actuators with each disclosed example configured to mitigate degradation of a site location at which a wearable drug delivery device is placed on a user's body.

In the disclosed examples, an example of a wearable drug delivery device is embedded with vibrational actuators that cause vibrations to emanate into the skin and tissue around the site where the wearable drug delivery device is located, thereby intermittently massaging the skin tissue with the intention of promoting site health. Benefits of regular massaging of an attachment location of a wearable drug delivery device may enhance blood flow beneath the wearable drug delivery and may reduce potential variation in insulin sensitivity over time at the wearable drug delivery device site. In addition, using ultrasonic vibration during removal of the wearable drug delivery device may reduce pain. In the examples, massaging by actuation of the vibrational actuators may be controlled by a controller that is included in a personal diabetes management device, a control application implemented on a smart device, or the wearable drug delivery device itself. The vibrational actuators may be configured to vibrate and emit vibrations over a range of vibration frequencies. The vibration frequency, or vibration frequencies, of the emitted vibrations may be selected by the controller from the range of vibration frequencies that the vibrational actuators are configured to output. The duration of the emitted vibrations may be from, milliseconds to seconds. The emissions of the vibrations, referred to herein as a vibrational event, to a site or location of attachment of a wearable drug delivery device are intended to increase blood circulation and enable better blood flow.

Examples of the wearable drug delivery device equipped with the vibrational actuators and processes for controlling managing the vibrational events are described in more detail in the following discussion with reference to the drawings.

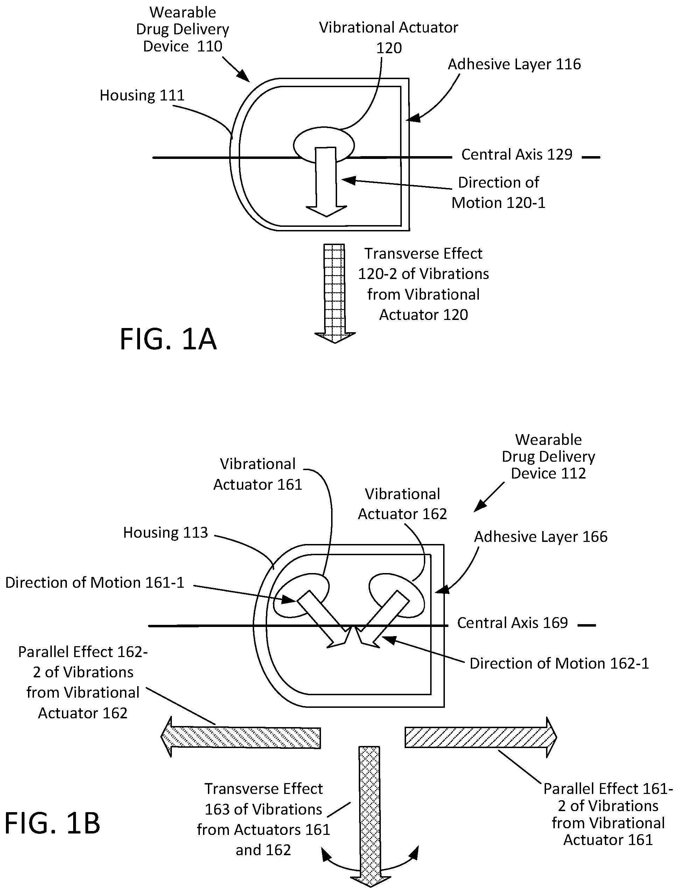

FIG. 1A shows an example of a wearable drug delivery device equipped with an example of a first type of vibrational actuator. In the example, the wearable drug delivery device 110 includes a housing 111, a vibrational actuator 120 and adhesive layer 116. The housing 111 may be multiple housings, or at least one housing, and may have housing/casing modifications that are configured to modulate with relative freedom to vibrate in the transverse/parallel direction into the skin surface with respect to the central axis 129. For example, the housing may be modified to selectively allow vibration in one direction compared to the another (e.g., solid structure versus hollow structure or solid versus ridged structures, or the like), or also have structural features that amplify the vibration in one or more directions (e.g., solid structure in one direction versus hollow or rigid structure in the other, or the like). The at least one housing 111 may also be configured to retain the vibrational actuator 120 as well as other devices and circuitry described with reference to later example. The housing 111 has a central axis 129.

The adhesive layer 116 may be a water-proof adhesive configured to affix the wearable drug delivery device 110 to the skin of a user of the wearable drug delivery device 110. The adhesive layer 116 may be coupled to the at least one housing 111 by adhesive, which may be the same as or different from that which affixes the wearable drug delivery device to the skin of the user, or some form of fastener (e.g., rivets, welds or the like). The adhesive layer 116 may be configured to minimize or limit the attenuation of the vibrations generated by the vibrational actuator 120.

The vibrational actuator 120 may be a piezo ceramic transducer that is configured to output vibrations in the direction of emission 120-1. The vibrational actuator 120 may be configured to output vibrations having a vibration frequency of approximately 1 MHz to approximately 10 MHz. The vibrational actuator 120 may be positioned within the at least one housing 111 of the wearable drug delivery device 110 to emit vibrations in a particular direction. The vibrations in the direction of emission 120-1 may be longitudinal waves or shear waves. Vibrations in the direction of emission 120-1 may create a transverse effect 120-2 in the skin of a user.

FIG. 1B shows another example of a wearable drug delivery device equipped with multiple vibrational actuators of the first type shown in FIG. 1A. The wearable drug delivery device 112 may include at least one housing 113, vibrational actuators 161 and 162, and adhesive layer 166. The at least one housing 113 has a central axis 169.

The adhesive layer 166 may be a water-proof adhesive configured to affix the wearable drug delivery device 112 to the skin of a user (shown in a later example) of the wearable drug delivery device 112. The adhesive layer 166 may be coupled to the at least one housing 113 by adhesive, which may be the same as or different from that which affixes the wearable drug delivery device to the skin of the user (shown in a later example), or some form of fastener (e.g., rivets, welds or the like). The adhesive layer 166 may be configured in a manner that minimizes or limits the attenuation of the vibrations generated by the vibrational actuators 161 and 162.

The vibrational actuators 161 and 162 may be piezo ceramic transducers that are configured to output vibrations having different vibration components. In an example, the respective vibrational actuators 161 and 162 are individually controllable to emit vibrations having respective directions of emission 161-1 and 162-1. In an example, vibrational actuator 161 may be individually controlled by a controller (described in more detail with reference to another example) to emit vibrations having the respective direction of emission 161-1. The effect of the vibrations in the direction of emission 161-1 may be a parallel effect 161-2 of the vibrational actuator 161 on the skin. Similarly, when vibrational actuator 162 may be individually controlled by the controller to emit vibrations, the emitted vibrations may have a respective direction of emission 162-1 within the skin. The effect of the vibrations on the skin in the direction of emission 162-1 may be a parallel effect 162-2 (i.e., parallel to the central axis 169). In addition, the vibrational actuator 161 and the vibrational actuator 162 may be controlled to emit vibrations in tandem. The result of the tandem generation of vibrations from the vibrational actuator 161 and the vibrational actuator 162 may be the transverse effect 163 (that is in a direction transverse to, or perpendicular to, the central axis 169) in the skin of the user. For example, the first vibrational actuator 161 and second vibrational actuator 162 may output a combination of vibrations that produce a combination of parallel effects 161-2 and 162-2 that may result in a transverse effect 163 that is not perpendicular to the central axis 169, but at an acute angle or an obtuse angle with respect to the direction from the central axis 169. In an example, via customized control signals, the intensity of the vibrations generated by the first vibrational actuator 161 may be greater than the vibrations generated by the second vibrational actuator 162 and as a result the direction of the transverse effect 163 may no longer be perpendicular to the central axis 169. Similarly, the intensity of the parallel effects 161-2 and 162-2 may also be manipulated based on the control signals applied to respective vibrational actuator 161 or 162.

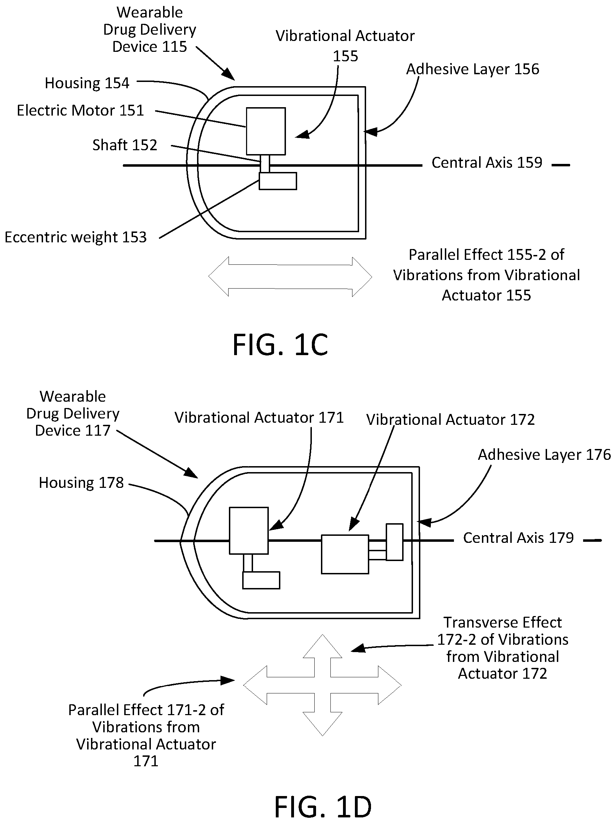

Types of vibrational actuators other than piezo ceramic transducers are also considered. FIG. 1C shows an example of a wearable drug delivery device equipped with an example of a second type of vibrational actuator. In the example of FIG. 1C, the wearable drug delivery device 115 includes at least one housing 154, a vibrational actuator 155, and an adhesive layer 156. The at least one housing 154 has a central axis 159.

The adhesive layer 156 may be a water-proof adhesive configured to affix the wearable drug delivery device 115 to the skin (shown in a later example) of a user of the wearable drug delivery device 115. The adhesive layer 156 may be coupled to the at least one housing 154 by adhesive, which may be the same as or different from that which affixes the wearable drug delivery device to the skin of the user, or some form of fastener (e.g., rivets, welds or the like). The adhesive layer 156 may be configured to minimize or limit the attenuation of the vibrations generated by the vibrational actuator 155.

The second type of vibrational actuator 155 may have a small electric motor 151 with a shaft 152 having an eccentric weight 153 on the shaft 152. The second type of vibrational actuator 155 may be configured to generate vibrations that have a vibration frequency that is lower than the vibration frequency of piezo ceramic transducer vibrational actuators. These second types of vibrational actuators are used in other types of devices, such as smart phones, tablets, toys, and the like. The vibrational actuator 155 may be positioned within the at least one housing 154 such that vibrations (that are longitudinal waves or shear waves) generated by the vibrational actuator 155 produce a parallel effect 155-2 in the skin that is perpendicular to the central axis 159 of the at least one housing 154. The magnitude of vibration produced by the vibrational actuator 155 may be affected by various factors, such as, for example, the speed of rotation of the eccentric weight 153, the mass of the eccentric weight 153, the distance of the eccentric weight 153 from the shaft 152, and the like. The vibrational frequency of vibrations generated by the second type of vibrational actuator may range from tens of Hz to approximately 300 Hz depending upon the applied voltage and available current.

FIG. 1D shows another example of a wearable drug delivery device equipped with multiple vibrational actuators of the second type shown in FIG. 1C. In the example of FIG. 1D, the wearable drug delivery device 117 includes a housing 178, a first vibrational actuator 171, a second vibrational actuator 172, and an adhesive layer 176. The housing 178 has a central axis 179.

The adhesive layer 176 may be a water-proof adhesive configured to affix the wearable drug delivery device 117 to the skin of a user (shown in a later example) of the wearable drug delivery device 117. The adhesive layer 176 may be coupled to the housing 178 by adhesive, which may be the same as or different from that which affixes the wearable drug delivery device to the skin of the user (shown in a later example), or some form of fastener (e.g., rivets, welds or the like). The adhesive layer 176 may be configured to minimize or limit the attenuation of the vibrations generated by the vibrational actuators 171 and 172.

The first vibrational actuator 171 and the second vibrational actuator 172 are each positioned with reference to the central axis 179 to produce different effects in the skin of a user when each is vibrating. For example, when vibrating, the first vibrational actuator 171 may produce vibrations in a direction parallel to the central axis 179 that produce a parallel effect 171-2 of vibration in the skin of the user. In contrast, the second vibrational actuator 172, when vibrating, may produce an effect, a transverse effect 172-2, of vibrations in the skin of a user from vibrational actuator 171 that is transverse to the central axis 179. In an example, a controller may be configured to modulate the speed of the electric motor of the respective vibrational actuators 171 and 172 to create changes in the vibration frequency as well as the direction of rotation (e.g., changing polarity of voltage applied to the electric motor) of the electric motor to create vibrations in different directions. As a result, the control of the combination of the electric motors of the respective vibrational actuators 171 and 172 at the two positions and opposing angles are configured to deliver variable vibration patterns that are combination of the parallel effects 171-2 and transverse effects 172-2 on the skin.

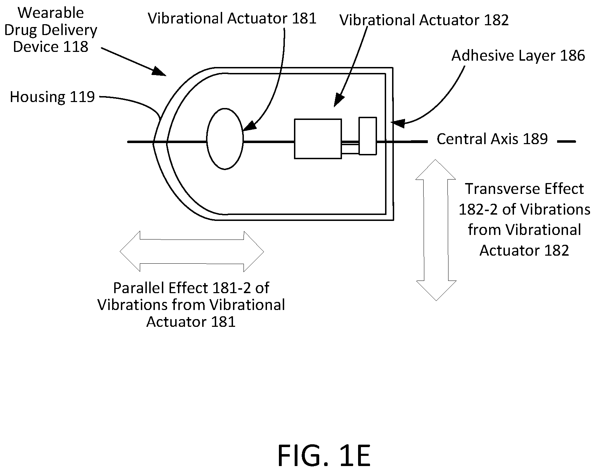

FIG. 1E shows a further example of a wearable drug delivery device equipped with a combination of first type and second type vibrational actuators such as those shown in the examples of FIGs. 1A and 1C. The wearable drug delivery device 118 may include at least one housing 119, vibrational actuator 181, vibrational actuator 182, and adhesive layer 186. The at least one housing 119 has a central axis 189.

The adhesive layer 186 may be a water-proof adhesive configured to affix the wearable drug delivery device 118 to the skin of a user (shown in a later example) of the wearable drug delivery device 118. The adhesive layer 186 may be coupled to the at least one housing 119 by adhesive, which may be the same as or different from that which affixes the wearable drug delivery device to the skin of the user (shown in a later example), or some form of fastener (e.g., rivets, welds or the like). The adhesive layer 186 may be configured to minimize or limit the attenuation of the vibrations generated by the vibrational actuators 181 and 182.

In the example, the vibrational actuator 181 may be a first type of vibrational actuator and vibrational actuator 182 may be a second type of vibrational actuator 182. Each respective vibrational actuator 181 and 182 may be positioned with respect to the central axis 189 of the housing 119 and may generate vibrations that cause different effects. For example, the vibrational actuator 181 may cause the parallel effect 181-2 in the skin of a user, which is vibrational effects that are parallel to the central axis 189 of the at least one housing 119. Alternatively, the vibrational actuator 182 may cause the transverse effect 182-2 in the skin of a user, which is vibrational effects that are transverse to the central axis 189 of the at least one housing 119.

The parallel effects, transverse effects and combined parallel and transverse effects of the vibrations generated by the vibrational actuators in the examples of FIGs. 1A-1E may be applied to the skin at an attachment location of a wearable drug delivery device on a user's body. It may be helpful to describe more details of the wearable drug delivery device.

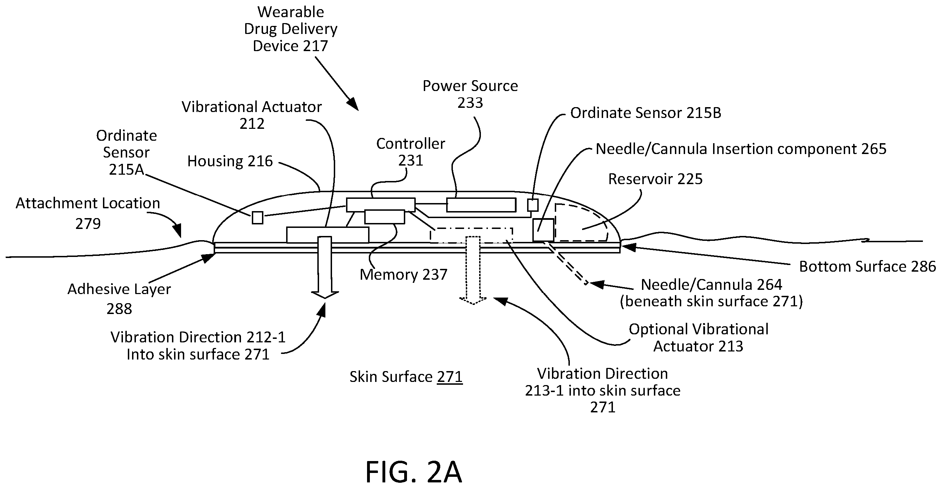

FIG. 2A illustrates a cross-sectional, functional block diagram of an example of a wearable drug delivery device that is suitable for implementing the example processes and techniques described herein.

In the example of FIG. 2A, the wearable drug delivery device 217 may be positioned at an attachment location 279 that is on the skin surface 271 of a user. The attachment location 279 may be, for example, a user's abdomen, lower back, upper arm, thigh, or other location that supports delivery and uptake of the liquid drug, such as insulin, provided by the wearable drug delivery device. Note the liquid drug is stored in a reservoir within the at least one housing 216, but, for ease of discussion, these detailed structures are shown in and discussed with reference to another example. The wearable drug delivery device 217 is affixed to the user's skin surface 271 via the adhesive layer 288 at the attachment location 279.

The attachment location 279 may be a location where the wearable drug delivery device 217 initiates a needle insertion process by which needle/cannula insertion component 265 is operated to insert needle/cannula 264 beneath the skin surface 271 of the user. The therapeutic drug contained in the reservoir 225 is delivered to the user via the needle/cannula 264 that is positioned beneath the skin surface 271. The vibrational actuators 212 and 213 may be disconnected from the needle/cannula 264 as well as the needle/cannula insertion component 265. For example, the vibrational actuators 212 and 213 may be spaced apart from the needle/cannula 264. In an example of such a configuration, neither the vibrational actuator 212 nor the vibrational actuator 213 do contact or touch the needle/cannula 264. In another example, the vibrational actuator may be directly coupled to the needle/cannula 264 to enhance delivery of the drug.

The wearable drug delivery device 217 may include at least one housing 216 and the adhesive layer 288 on a bottom surface 286 of the housing 216. The adhesive layer 288 may extend around a perimeter of the at least one housing 216, for example, on the bottom surface 286. The at least one housing 216 may be configured to contain vibrational actuator 212, controller 231, memory 237, power source 233 and a reservoir 225. In an additional example, the housing may also be configured to contain ordinate sensors 215A and 215B. The reservoir 225 may be configured to store a therapeutic drug, such as insulin, morphine, or the like. The at least one housing 216 may include a bottom surface 286 to which is disposed an adhesive layer 288. The adhesive layer 288 may be configured to affix the at least one housing to a skin surface 271 of a user.

In an example, the power source 233 may be coupled to the vibrational actuator 212 (and optional vibrational actuator 213 depending upon the implementation) and to the controller 231. The power source 233 may be an internal battery, a supercapacitor or another suitable form of electrical energy storage device. The internal battery may be rechargeable through induction or through energy harvesting techniques as an example. Alternatively, the power source 233 may be an energy harvesting device, such as a coil spring, a piezoelectric device, a thermoelectric device (e.g., generating electricity based on temperature gradients), a light conversion device, a kinetic energy device, or the like, that is configured to harvest enough power, for example, from body motion, light or the like to generate vibrations upon receipt of control signals from the controller 231. The power source 233 may be a combination of both a battery and an energy harvesting system.

The memory 237 may be coupled to the controller 231 and configured to store programming code, site maintenance application, and settings, wherein the programming code and site maintenance application are executable by the controller 231. The site maintenance application (described in more detail with reference to another example) may configure the controller 231 to perform different functions with regard to controlling the vibrational actuator 212 and, if equipped, vibrational actuator 213.

The controller 231 may be configured to receive signals, for example, from the ordinate sensors 215A and 215B. The ordinate sensors 215A and 215B may be configured to output a signal indicative of their position, such as roll, attitude and yaw, which may be usable to determine the site location 279 on the body of a user. For example, ordinate sensors 215A and 215B positioned at an upper, right arm of a user may generate different signals indicative of roll, attitude and yaw than the signals generated when the wearable drug delivery device is placed, for example, on the right side of the abdomen of a user.

The controller 231 may be further configured to output a modulated control signal to the vibrational actuator 212, and in response the modulated control signal, the vibrational actuator 212 may be configured to generate modulated vibrations according to the modulated control signal.

In an example, the wearable drug delivery device 217 may be equipped with a single vibrational actuator, such as 212. In another example, the wearable drug delivery device 217 may be equipped with a multiple vibrational actuators, such as vibrational actuator 212 and optional vibrational actuator 213.

In an example, the vibrational actuator 212 may be positioned within the at least one housing 216 to transmit vibrations that are either transverse or parallel to a central axis of the at least one housing 216. In a further example, the vibrational actuator may be a piezo ceramic transducer controllable to transmit vibrations having a frequency between approximately 1 MHz and approximately 10 MHz.

Examples of vibrational actuators, such as 212 and 213, suitable for use in the wearable drug delivery device 217 include piezo electric transducers that generate ultrasonic vibrations that are intended to reduce swelling, edema and soften scars. For example, the vibrational actuators may be oscillated at a frequency between 1-10 MHz. Ultrasonic transducers can be oscillated at much higher frequencies (MHz) compared to the electric motors (~ 300 Hz). The oscillating ultrasonic transducer of the vibrational actuator causes vibrations within the housing 216 that are emitted through the bottom surface 286 of the housing 216 to the skin surface when the housing is affixed (e.g., at attachment location 279) to a user. The piezo ceramic transducers in a coin shaped disc form factor that generate vibrations at resonant frequencies in the range of approximately 1 MHz to approximately 3 MHz, or approximately 2 MHz to approximately 10 MHz. Alternatively, the vibrational actuator 212, or the optional actuator 213, may configured to generate vibrations at a substantially fixed resonant frequency, such as 2.5 MHz or the like.

In another example, the vibrational actuator 212 may be an electric motor having a shaft and an eccentric weight coupled to the shaft. The vibrational actuator 212 is controllable to transmit vibrations having a frequency range of tens of HZ to hundreds of Hz. A frequency range of, for example, approximately 20 Hz to approximately 300Hz may provide sufficient stimulation to an attachment location to provide a benefit.

In an operational example, the vibrational actuator 212 may be coupled to the controller 231. The controller 231 may be configured to output control signals, for example, to the vibrational actuator 212. The vibrational actuator 212 may be configured to generate vibrations in response to a control signal from the controller 231. For example, the controller 231 may be operable to output control signals that cause the vibrational actuator to generate vibrations at select frequencies or selected range of frequencies. In an example, the controller 231 may be configured to cause the vibrational actuator 212 (and optional vibrational actuator 213) to generate vibrations. The vibrations generated by the respective vibrational generator 212 or 213 may have a duration, such as 0.1 second, 0.5 seconds, 1 second, 3 seconds, or the like, and a frequency, e.g., 100-300 MHz, 1-100Hz, a combination of different frequencies, or the like. The vibrations generated by the vibrational actuator 212 (as well as optional vibrational actuator 213) may be emitted through the bottom surface 286 of the at least one housing 216 through the adhesive layer 288 to be applied to and extend below the skin surface 271.

The vibrations applied to the skin surface 271 may be composed of wave components in directions transverse to a central axis (shown in an earlier example) of the housing 216 of the wearable drug delivery device 217, wave components in directions parallel to the housing 216 of the wearable drug delivery device 217, or a combination of transverse wave components and parallel wave components that extend into the skin surface 271 (as shown in earlier examples).

In a further example that utilizes the optional vibrational actuator 213, the vibrational actuator may be a first vibrational actuator, such as 212, and a second vibrational actuator, such as optional vibrational actuator 213. In the further example, the first and second vibrational actuators may be the same type of vibrational actuator (e.g., both are piezo ceramic transducers, or both are electric motors with the eccentric weights).

In an alternative example, the first vibrational actuator, such as 212, may be a piezo ceramic transducer and the second vibrational actuator, such as 213, may be an electric motor having a shaft and an eccentric weight coupled to the shaft of the electric motor. In the alternative example, the first vibrational actuator 212 may be positioned within the at least one housing 216 to transmit vibrations, when actuated, in a first direction that is substantially parallel to a central axis of the housing 216. Similarly, the second vibrational actuator 213 may be positioned within the housing to transmit vibrations, when actuated, in a second direction that are also substantially parallel to the central axis of the at least one housing 216. In the alternative example, the first direction may be opposite to the second direction and the first vibrational actuator 212 and second vibrational actuator 213 may be alternately actuated. The vibrations generated by respective vibrational actuators 212 and 213 may be applied to and enter the skin surface 271 as described with reference to in the examples of FIGs. 1A-1E as transverse and parallel waves.

In an example, the vibrational actuators 212 and 213 may be positioned about the central axis of the at least one housing 216 as shown in the example of FIG. 1B. When the vibrational actuators 212 and 213 are positioned in substantially the same positions as vibrational actuators 161 and 162 in FIG. 1B within at least one housing 216, the controller 231 may be configured to simultaneously control actuation of the first vibrational actuator 212 and the second vibrational actuator 213 to transmit vibrations in a direction that is transverse to the central axis of the at least one housing 216.

In an operational example, the controller may be further configured to perform different functions. For example, the controller 231 may be configured to monitor a power reserve of the power source 233. The power reserve may be an amount of electrical power that remains in the power source 233 whether the power source 233 is a battery or an energy harvesting device. The controller 231 is further configured to determine, based on a result of the monitoring of the power reserve of the power source, a time interval between actuations of the vibrational actuator 212.

In an operational example, the controller may periodically actuate the vibrational actuator(s) 212 (and 213) at various intervals. For example, the vibrational actuator(s) 212 (and 213) may be actuated every hour, every 2 hours or the like. The time interval between actuations is a period of time from when a vibrational actuator is actuated to deliver vibrations until a subsequent actuation of the vibrational actuator to again deliver vibrations.

The vibrations generated by the vibrational actuator(s) 212 (and 213) may enter into the skin surface 271 as shown by vibration directions 212-1 and 213-1, respectively.

Examples of periods of time that may be a time interval include 1 hour, 2 hours, 6 hours, 12 hours and the like. In a further example, the controller, when determining the time interval between actuations of the vibrational actuator, may compare the result of the monitoring of the power reserve of the power source 233 to a power reserve threshold. The result of the monitoring of the power reserve may be a voltage or current that indicates an amount of reserve power. For example, the monitoring of the power reserve may indicate 12 hours of reserve power of the power source 233 in the wearable drug delivery device 217 that has been in use, for example, for 60 hours of its 72-hour lifetime. The power reserve threshold may be a value that is based on the number of hours that a wearable drug delivery device has remaining in its lifecycle.

Wearable drug delivery devices, such as 217, have a lifecycle that is typically 72 hours or 3 days. At the end of the lifecycle, the power source 233 is expected to have a minimal reserve, such as 1-6 hours, 2-6 hours, less than 10%, 5-10% or the like, depending upon the diabetes treatment plan of the user. The power reserve threshold may vary from user to user and also between different times of year, such as June to August as compared to January to March, or the like, for example, depending upon how often the wearable drug delivery device is used. The controller may be configured to modify the power reserve threshold based on usage patterns of the wearable drug delivery devices by the particular user. In addition, the wearable drug delivery device may generate an alarm when the power reserve threshold is exceeded or within a range that the controller may avoid actuating the vibrational actuators.

Returning to the example, based on the power reserve being below a preset power reserve, the controller 231 may output a signal causing a schedule of vibrational events to be modified. A vibrational event is when a controller outputs a control signal to actuate a vibrational actuator until the vibrational actuator ceases to deliver vibrations in response to the control signal. Various settings of the vibrational event may be maintained in the schedule of vibrational events (described in more detail with reference to another example). The various settings of the vibrational event may be duration of vibrational event (e.g., milliseconds to seconds), timing of vibrational event (e.g. 20 minutes after installation, 2 minutes prior to administering a bolus, 20 minutes before set bed time, every hour during waking hours, or the like), vibration effect (e.g., parallel or transverse), a vibration frequency (e.g., a default frequency of the respective vibrational actuator, a selectable frequency, frequency that is a result of a modulated control signal for the duration of the event), and the like. In an example, each vibrational event may have a preset duration and a preset vibration frequency as settings of the respective vibrational event.

The schedule of vibrational events may be preset vibrational events that are separated by time intervals for application by the controller. The vibrational event schedule may be set for the lifecycle of every wearable drug delivery device for the user based on user history and user need for application of vibrational events. In some examples, the schedule of vibrational events may be based on an attachment location, such as 279, of the wearable drug delivery device 217.

The ordinate sensors 215A and 215B may be located a fixed positions within the at least one housing 216 of the wearable drug delivery device 217. The respective ordinate sensors 215A and 215B may be an inertial sensor, a gyroscope or the like that may be used to determine an orientation of the wearable drug delivery device 217 and a likely location of the wearable drug delivery device on the user's body. Each ordinate sensor 215A and 215B may, for example, be configured to output a signal indicating an orientation of the respective ordinate sensor.

In an additional example, the controller may be further configured to receive a signal output from each ordinate sensor 215A and 215B. Using information contained in the signal output from each respective ordinate sensor 215A and 215B, the controller 231 may determine an attachment location 279 (e.g., thigh, upper arm, lower back, abdomen, or the like) of the wearable drug delivery device 217 on a user. The controller 231 may output an indication of the attachment location to cause selection of a vibrational event schedule. A vibrational event schedule may be a list of all vibrational events that are to be administered to a user over a number of hours, a day, several days, a lifecycle of a wearable drug delivery device, or the like.

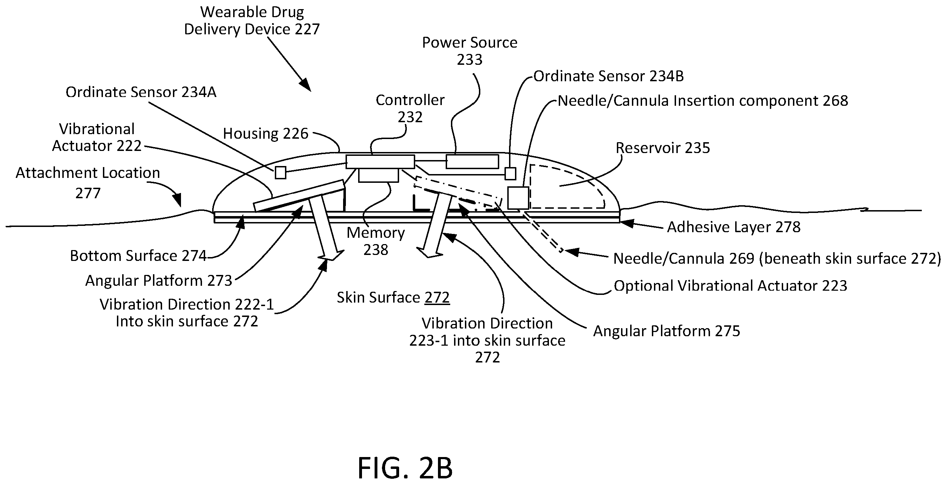

Other examples related to the modifications of the positioning of the vibrational actuators within the housing(s) of the wearable drug delivery devices are also contemplated. FIG. 2B illustrates another cross-sectional structural example and functional block diagram of a wearable drug delivery device example.

In the wearable drug delivery device example of FIG. 2B, the wearable drug delivery device 227 includes at least one housing 226, a controller 232, memory 238, a power source 233, vibrational actuator 222, and reservoir 235. In another example, the wearable drug delivery device 227 also includes optional vibrational actuator 223 and ordinate sensors 234A and 234B. The at least one housing 226 may include a bottom surface 274 and an adhesive layer 278. The adhesive layer 278 may be beneath the bottom surface 274 and may be configured to adhere to the skin surface 272 of a user. The bottom surface and the adhesive layer 278 may be configured to transmit vibrations generated by the vibrational actuator 222 and, when present, the optional vibrational actuator 223.

The wearable drug delivery device 227 may be positioned at attachment location 277 on the skin surface 272 of the user. The attachment location 277 may be a location where the wearable drug delivery device 227 initiates a needle insertion process by which needle/cannula insertion component 268 is operated to insert needle/cannula 269 beneath the skin surface 272 of the user. The therapeutic drug contained in the reservoir 235 is delivered to the user via the needle/cannula 269 that is positioned beneath the skin surface 272. The vibrational actuators 222 and 223 are uncoupled from the needle/cannula 269 as well as the needle/cannula insertion component 268.

The memory 238 may be coupled to the controller 232 and configured to store programming code, site maintenance application, and settings, wherein the programming code and site maintenance application are executable by the controller 232. The site maintenance application (described in more detail with reference to another example) may configure the controller 232 to perform different functions with regard to the controlling the vibrational actuator 222 and, if equipped, vibrational actuator 223. The memory 238 may store a vibrational event schedule.

Angular platforms 273 and 275 may be structural modifications to the wearable drug delivery device 217 in the example of FIG. 2A. The angular platforms 273 and 275 may be formed from material or materials that provide maximum transmission of the vibrations generated by the respective vibrational actuators 222 and 223. The angular platforms 273 and 275 may enable the vibrational actuators 222 and 223 to be angled, for example, 20-65 degrees with respect to the bottom surface 274 of the at least one housing 226. In a specific example, the angular platforms 273 and 275 may provide a 45-degree angle. Due to the presence of the angular platform 273, the vibrational actuator 222 is positioned at an angle with respect to the bottom surface 274. The vibrational actuator 222 may be coupled to the angular platform 275 and may transmit vibrations through the angular platform 275 actuator, the bottom surface 274 and the adhesive layer 278. The angular platforms 273 and 275 may cause the vibration directions 222-1 and 223-1 to be angled from the central axis (not shown in this example) and with reference to the skin surface 272. The vibrations generated by respective vibrational actuators 222 and 223 and emitted from the respective angular platforms 273 and 275 may be applied to the skin surface 272 as shown by vibration directions 222-1 and 223-1. For example, the vibrations applied to the skin surface 272 may be composed of wave components in directions transverse to a central axis (shown in an earlier example) of the at least one housing 226 of the wearable drug delivery device 227, wave components in directions parallel to the at least one housing 226 of the wearable drug delivery device 227, or a combination of transverse wave components and parallel wave components that extend into the skin surface 272 (as shown in earlier examples).

The vibrational actuators 222 and 223 may be disconnected from the needle/cannula 269 as well as the needle/cannula insertion component 268. For example, the vibrational actuators 222 and 223 may be spaced apart from the needle/cannula 269. In an example of such a configuration, neither the vibrational actuator 222 nor the vibrational actuator 223 contact or touch the needle/cannula 269. In another example, the vibrational actuators 222 and 223 may be directly coupled to the needle/cannula 269 to enhance delivery of the drug.

The examples of the wearable drug delivery device as shown herein with respect to the examples of FIGs. 1A-2B are configured so vibration waves (i.e., transverse, parallel or a combination of both) generated by the vibrational actuators enter the body through the skin surface as shown in the respective examples.

The controller 232 may be operable to perform various functions (e.g., control of the vibrational actuators and delivery of insulin) based on programming code stored in the memory 237. For example, the angular platform 273 may be configured to output the vibrations output from vibrational actuator 222 at an angle of approximately 20-65 degrees and the controller 232 may be configured to modulate controls signals applied to the vibrational actuator 222 to affect a massage in the area the attachment location 277 by manipulating the vibration direction 222-1. The area to which the massage is effective may be within several centimeters around the attachment location 277 (e.g., approximately 1-5 centimeters from the center of the at least one housing 226) that may emanate in over 360 degrees along and within the skin surface 272. Similarly, the vibrational actuator 223 may also be controlled to affect a massage in the area the attachment location 277 by manipulating the vibration direction 223-1. Examples of the control of the vibrational actuators 222 and 223 are described with reference to other examples.

While the controller 232 may control the vibrational actuators 222 and 223 differently than vibrational actuators 212 and 213 of FIG. 2A due to the presence of the angular platforms 273 and 275, other functions performed by the controller 232 may be similar to those performed by controller 231 of the example of FIG. 2A. The other components of wearable drug delivery device 227 may perform similar functions as the similar components of wearable drug delivery device 217. For example, the ordinate sensors 234A and 234B may output signals indicative of an attachment location 277 that may be utilized by the controller 232 to determine the attachment location 277 on the user's body.

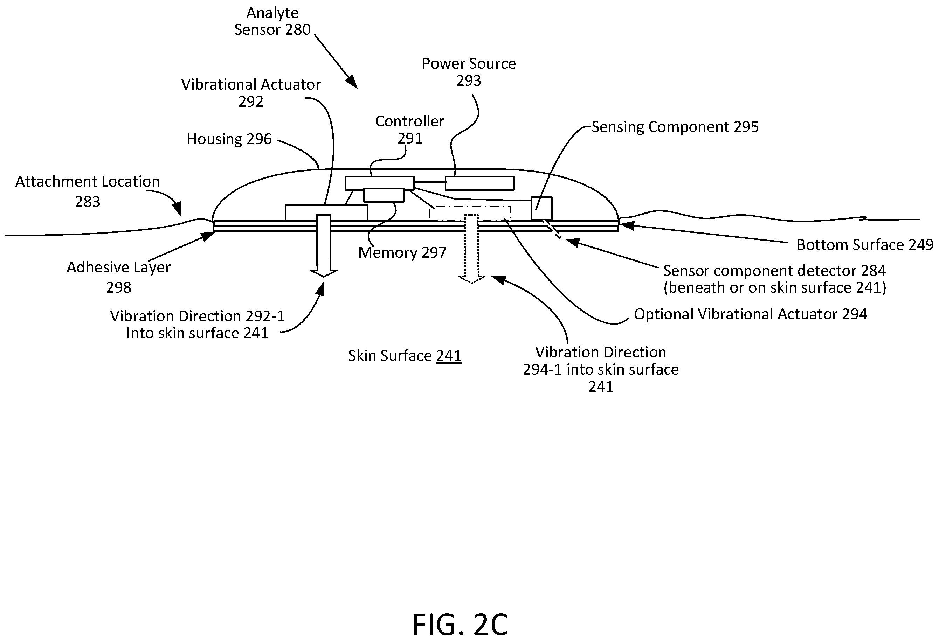

Other wearable devices may also benefit from the vibrational therapy or massage provided by the devices described with reference to the described examples of FIGs.1A-2B, such as an analyte sensor or the like. FIG. 2C illustrates a cross-sectional, functional block diagram of an example of a wearable analyte sensor that is suitable for implementing the example vibrational processes and techniques described herein.

In the example of FIG. 2C, the analyte sensor 280 may be positioned at an attachment location 283 that is on the skin surface 241 of a user and may be positioned at a location away from the wearable drug delivery devices described with reference to FIGs 2A and 2B. The attachment location 279 may be, for example, a user's abdomen, lower back, upper arm, thigh, or other location that supports delivery and uptake of the liquid drug, such as insulin, provided by the wearable drug delivery device. The analyte sensor may be affixed to the user's skin surface 241 via the adhesive layer 298 at the attachment location 283.

The attachment location 283 may be a location where the analyte sensor is operable to detect one or more different types of analytes using a sensor component detector 284 that may be implemented to penetrate a portion of the skin surface 241 or be placed on the skin surface 241, or a combination of both. The sensor component detector 284 may be a tube(s), a material or materials that are reactive to different analytes and may provide data to the sensing component 295.

The analyte sensor 280 may include at least one housing 296 and the adhesive layer 298 on a bottom surface 249 of the at least one housing 296. The at least one housing 296 may include a bottom surface 286 to which is disposed an adhesive layer 288. The adhesive layer 288 may be configured to affix the housing to a skin surface 271 of a user. The adhesive layer 298 may extend around a perimeter of the at least one housing 296, for example, on the bottom surface 249. The at least one housing 296 may be configured to contain vibrational actuator 292, controller 291, memory 297, and power source 293.

In an example, the power source 233 may be coupled to the vibrational actuator 212 (and optional vibrational actuator 213 depending upon the implementation) and to the controller 231. The power source 233 may be an internal battery, a supercapacitor or another suitable form of electrical energy storage device. The internal battery may be rechargeable through induction or through energy harvesting techniques as an example. Alternatively, the power source 293 may be an energy harvesting device, such as a coil spring, a piezoelectric device, a thermoelectric device (e.g., generating electricity based on temperature gradients), a light conversion device, a kinetic energy device, or the like, that is configured to harvest enough power, for example, from body motion, light or the like to generate vibrations upon receipt of control signals from the controller 291. The power source 293 may be a combination of both a battery and an energy harvesting system.

The memory 297 may be coupled to the controller 291 and configured to store programming code, site maintenance application, and settings, wherein the programming code and site maintenance application are executable by the controller 291. The site maintenance application (described in more detail with reference to another example) may configure the controller 291 to perform different functions with regard to controlling the vibrational actuator 292 and, if equipped, vibrational actuator 294.

The vibrational actuators 292 and 294 may be configured as described with FIGs. 1A-2B. Examples of vibrational actuators, such as 292 and 294, suitable for use in the analyte sensor 280 include piezo electric transducers that generate ultrasonic vibrations that are intended to alleviate pain during removal of the analyte sensor as well as reduce swelling or the like. For example, the vibrational actuators may be oscillated at a frequency between 1-10 MHz. Ultrasonic transducers can be oscillated at much higher frequencies (MHz) compared to the electric motors (~ 300 Hz). The oscillating ultrasonic transducer of the vibrational actuator causes vibrations within the housing 296 that are emitted through the bottom surface 249 of the housing 816 to the skin surface when the housing is affixed (e.g., at attachment location 283) to a user. The piezo ceramic transducers in a coin shaped disc form factor that generate vibrations at resonant frequencies in the range of approximately 1 MHz to approximately 3 MHz, or approximately 2 MHz to approximately 10 MHz. Alternatively, the vibrational actuator 292, or the optional actuator 294, may be configured to generate vibrations at a substantially fixed resonant frequency, such as 2.5 MHz or the like.

The vibrations generated by the vibrational actuator(s) 292 (and 294) may enter into the skin surface 241 as shown by respective vibration directions 292-1 and 294-1.

The vibrational actuators 292 and 294 may be disconnected from the needle/cannula 269 as well as the sensor component 265 or sensor component detector 284. For example, the vibrational actuators 292 and 294 may be spaced apart from the sensor component 265 or sensor component detector 284. In an example of such a configuration, neither the vibrational actuator 292 nor the vibrational actuator 294 do contact or touch either the sensor component 265 or the sensor component detector 284. In another example, the vibrational actuator 292 or 294 may be directly coupled to the sensor component detector 284 to enhance the accuracy of the analyte sensor 503.

Control and coordination of the vibrational actuator 292, or the optional actuator 294, may be by the controller 291 and programming code or setting stored in the memory 297. The operation or coordination of the respective vibrational actuators 292 and 294 may be performed as described with reference to the examples of FIGs. 1A-2B. An analyte sensor, such as 280, would be beneficial because frequent vibration around the analyte sensor 280 may enhance the accuracy and also reduce skin irritation at the attachment location 283.

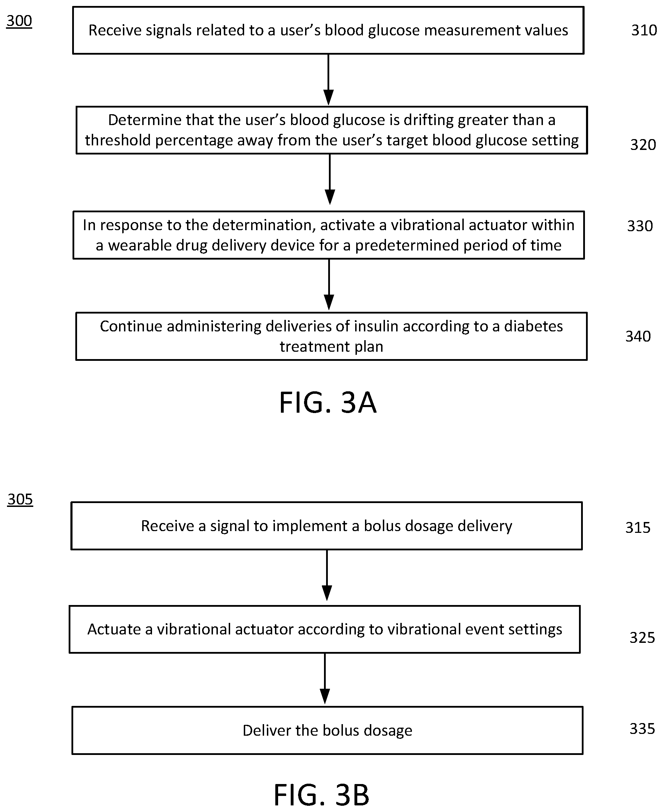

FIG. 3A shows a flow chart of an example process for actuating a vibrational actuator within a wearable drug delivery device.

In the process 300, an analyte sensor may measure an analyte of a user over a period of time and provide measurements related to an analyte of the user to the wearable drug delivery device. In an example, the analyte sensor may be a blood glucose monitor that may measure a user's blood glucose over time and send signals indicating blood glucose measurement values to a management device and/or other devices. As discussed with reference to a later example, the management device or the other device may be a smartphone, a tablet, a dedicated personal diabetes management device, or the like. The signals sent by the blood glucose monitor may also indicate a trend (e.g., upward or downward) of historical blood glucose measurement values. The historical blood glucose measurement values may be values from blood glucose measurements made by the blood glucose monitor over, for example, the past ten blood glucose measurements or the blood glucose measurements made over the past 2, 3, 12 or 24 hours, or some other preset or default period of time.

At 310, the signal or signals, whether the indications of the blood glucose measurement values, the trend blood glucose measurement values, or both, may be received by a processor executing the process 300.

An application, such as an artificial pancreas application, may provide information to the site management application determine that the user's blood glucose is drifting greater than a threshold setting. The threshold setting may be a percentage, such as 10%, 20% or the like, above a user's target blood glucose setting. Alternatively, the threshold setting may be a set or default blood glucose value away from the user's target blood glucose setting (320). The processor executing programming code may, in response to the determination, activate a vibrational actuator within a wearable drug delivery device for a predetermined period of time (330). For example, the processor may cause a command signal to be sent to a controller on the wearable drug delivery device indicating that the controller is to initiate a vibrational event (e.g., a set duration, set vibrational frequency or frequencies, and the like). The vibrational event may have a duration as indicated in the command signal. Alternatively, the vibrational event may have a default duration, default frequency and the like. Deliveries of insulin may continue to be administered according to a diabetes treatment plan (340). The delivery of insulin may occur before, during or after the vibrational event.

In another example of actuating a vibrational event, FIG. 3B illustrates a flowchart for actuating a vibrational actuator when a bolus dosage is indicated to be delivered. In this example, the controller on the wearable drug delivery device may be configured to actuate the vibrational event in response to bolus dosage delivery signals from the management device.

Prior to process 305, a management device may determine that a bolus dosage is to be delivered to the user. For example, the user may have just consumed a meal, or a need for a correction bolus may have been determined by an AP application executing on a management device, such as a personal diabetes management device, a smart accessory or a smartphone. The management device processor may send a command signal to the controller of the wearable drug delivery device. The command signal may include information indicating a vibrational event is to accompany the delivery of the bolus dosage. For example, the command signal may contain multiple bytes of data, and one of the bytes or at least a portion of the command signal may indicate that (e.g., when and/or how) a vibrational event is to be administered. The controller of the wearable drug delivery may receive the command signal and interpret the information in the signal to administer a vibrational event as well as administering bolus dosage (315).

The controller in response to receiving the command signal to deliver the bolus dosage and administer the vibrational event, may be configured, at 325, to actuate a vibrational actuator according to vibrational event settings, such as duration and frequency. The vibrational event settings may be either default or user established settings stored in a memory of the wearable drug delivery device. Alternatively, the command signal may include the vibrational event settings as part of the information in the command signal. The controller may actuate the vibrational actuator by outputting a control signal. In another alternative, the controller may be configured to administer a vibrational event upon receipt of a command signal that instructing the controller to deliver the bolus dosage.

The controller may deliver the bolus dosage according to the command signal (335). The command signal may include the timing of the vibrational event with respect to the timing of the delivery of the bolus dosage. Alternatively, the timing of the vibrational event with respect to the timing of the delivery of the bolus dosage may be a default setting that is stored in the memory of the wearable drug delivery device. Based on either the timing in the command signal or in the default setting, the controller may be configured to administer the bolus dosage before, during or after the vibrational event.

In the examples, a management device may be one or more of a personal diabetes management device, a smartphone, a smart accessory device (e.g., a smartwatch or other smart wearable device) may be configured to determine settings to implement a vibrational event for the wearable drug delivery device.

In a specific example, a number of vibrational event schedules may be stored in a memory. These may be default vibrational event schedules that are based on general user attributes, such as age, sex, physical activity level, meal schedules, insulin sensitivity, number of years using a wearable drug delivery device, and the like.

When a wearable drug delivery device is attached to a user and initialized for use, a vibrational event schedule from the number of vibrational event schedules may be selected for actuation by the controller of the wearable drug delivery device. The selection of the vibrational event schedule may be performed by site management code or an AP application of a management device or, by a user, who selects a vibrational event schedule from a list on a graphical user interface that shows the number of vibrational event schedules.

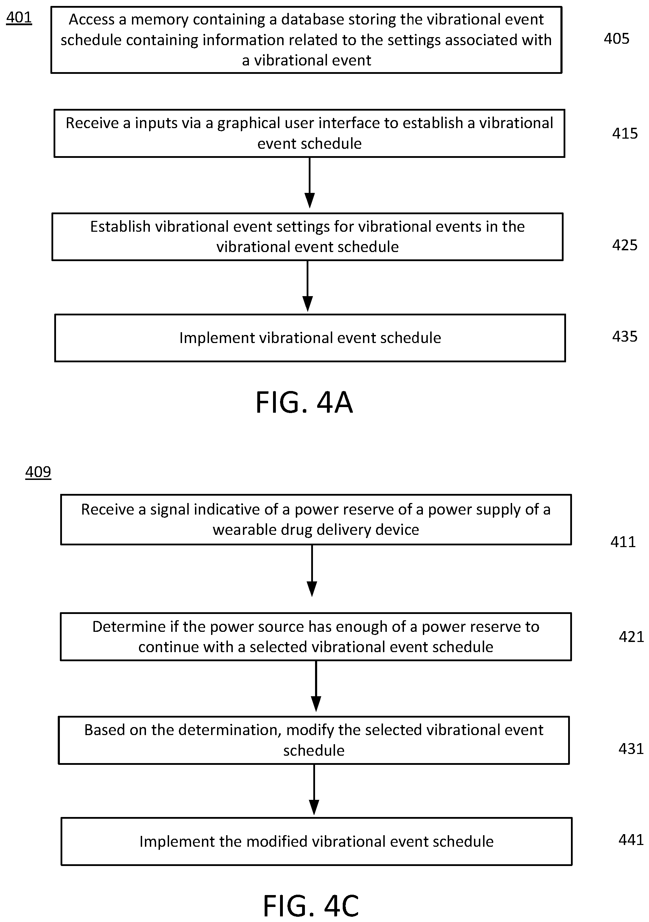

It may be helpful to describe an example that utilizes graphical user interface on a management device that enables users to generate or modify vibrational event settings as well as a vibrational event schedule for administering the vibrational events in the vibrational event schedule. FIG. 4A illustrates a flow chart of an example process for establishing a vibrational event schedule.

In the example process 401 of FIG. 4A, a management device may be configured to access a memory containing a database storing the vibrational event schedule containing information related to the settings associated with a vibrational event that is to be implemented on the wearable drug delivery device (405). The information related to the settings includes user preferences related to the vibrational event, wearable drug delivery device sensor indications, or time of day; and related information, such as intervals of when the vibrational event may be administered, particular times or occasions when a vibrational event is triggered or initiated, a frequency at which the vibrational actuator emits vibrations, a modulation of a control signal applied to the vibrational actuator, a duration of the vibrational event including a duration of the modulation of the control signal applied to the vibrational actuator. Information obtained from the database may be presented in a graphical user interface of a management device.

In the process 401, the vibrational event schedule may be established through user inputs to the graphical user interface presented on a display device, such as a touchscreen display device, of a management device (415). For example, one or all of the personal diabetes management device, the smartphone, and the smart accessor device described with reference in another example may be equipped with a touchscreen display device controlled by a processor within each respective device. Alternatively, a default vibrational event schedule may be initially set and subsequently modified. In an example, confirmation of setting of the default vibrational event schedule may be a response to a prompt presented on the graphical user interface.

The vibrational event schedule, whether the default vibrational event schedule or a custom vibrational event schedule set by user input, may have a number of different settings that are configurable by a user. Setting the vibrational event schedule may include modifying a default vibrational event schedule a modifying a previously-established vibrational event schedule or setting a new custom vibrational event schedule. In the example of FIG. 4A, the vibrational event settings for the vibrational events in the vibrational event schedule may be established at 425 via inputs to the graphical user interface.

In the example of a default vibrational schedule, the default vibrational schedule may be modified over time by user inputs modifying settings of the vibrational event schedule and to the vibrational events themselves. In addition, or alternatively, the default vibrational schedule may be automatically modified by the processor executing the AP application or site management application based on user history related to blood glucose measurement values, bolus delivery, mealtimes, physical activity, wearable drug delivery device power source status, drug delivery fluid pathway pressure settings, the time it takes for a bolus delivery to be completed, and the like. An example of drug delivery fluid pathway pressure settings may be based on an amount of pressure detected by device sensors, such as a pressure sensor, power source/energy harvesting circuit sensor, or the like. If a detected pressure within the fluid pathway (e.g., from reservoir to cannula) exceeds a certain threshold, as established in the pressure settings, then a vibrational event may be automatically triggered in an effort to reduce the amount of pressure within the fluid pathway, which may be indicative of an occlusion and/or poor drug delivery.

Examples of the user preferences that may be set or modified enable scheduling of a vibrational event at specific times during a day and selection of specific vibrational event settings. For example, the graphical user interface may enable scheduling of the vibrational event includes selection of daylight hours for administering the vibrational event, selection of evening hours for administering the vibrational event, or a combination of both daylight and evening hours. For example, the vibrational event may only be scheduled during times that a user is awake, so vibrational events may not be scheduled during the evening hours. In addition, the number of vibrational events may be set, and the site management application may be enabled to administer the vibrational events based on the set number of vibrational events.

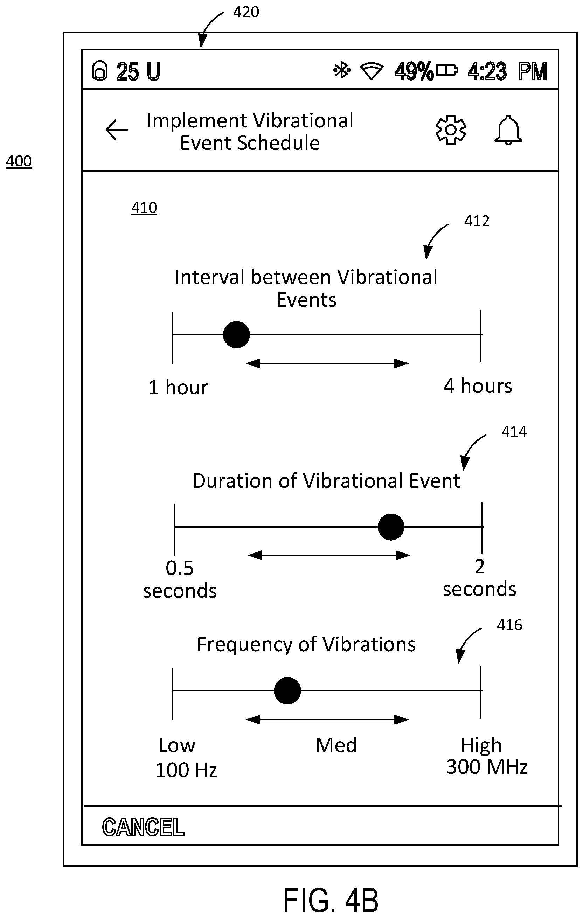

An example of a graphical user interface for setting the vibrational events is shown in FIG. 4B. FIG. 4B illustrates an example of a graphical user interface presented on an example management device as described herein. The management device 400 may include touchscreen display device 420 and a graphical user interface 410. The graphical user interface 410 may be presented by a processor executing a site management application (shown and described with reference to another example) of the management device 400. For ease of description only a few of the user preference settings for a vibrational event are shown in the example of FIG. 4B. The graphical user interface 410 may present a user input 412 directed to setting the interval between vibrational events. A slider or other input device may be used to allow a user to enter a setting. In the example, the interval between vibrational events may be set from 1 hour to 4 hours. Of course, other times may be presented and selected such as 30 minutes, 6 hours or the like. An additional setting includes a frequency of vibrations, for example, from a low vibration frequency (e.g., 100 Hz) to a high vibration frequency (e.g., 300 MHz) and a duration of the vibrational event, such as 1 second, 10 milliseconds, 20 milliseconds, 500 milliseconds or the like. At user input 414, the user may set the duration of the vibrational event from 0.5 seconds to 2 seconds. Of course, other times may be selected such as 0.1 seconds to 5 seconds or the like may be presented and selected.

In the example of FIG. 4B, a user input 416 may be used to set the frequency of vibration. In the user input 416, the user may select from a low, a medium or a high frequency. The low, medium or high frequency range may be different depending upon the vibrational actuators within the wearable drug delivery device. For example, in a wearable drug delivery device equipped with piezo ceramic transducers, the low frequency range may be approximately 1 MHz to less than 4 MHz, the medium frequency range may be approximately 4 MHz to less than 8 MHz, and the high frequency range may be approximately 8-10 MHz. Alternatively, if the wearable drug delivery device equipped with an electric motor and eccentric weight vibrational actuator, the low frequency range may be 1 Hz to less than 100 Hz, the medium frequency range may be approximately 100 Hz to less than 200 Hz, and the high frequency range may be approximately 200-300 Hz. The frequency of the vibrations may also be customized via user input 416. The low frequency may be set at approximately 100 Hz, which assumes that the wearable drug delivery device being controlled is equipped with a vibrational actuator having an electric motor and eccentric weight or other vibrational actuator capable of vibrating at the selected frequency. The high setting may be approximately 300MHz which assumes that the wearable drug delivery device being controlled is equipped with a vibrational actuator having a piezo ceramic transducer or the like. Of course, in an example, the presented frequencies 100Hz and 300 MHz may not be presented and only the low, medium and high settings may be presented to avoid confusing the user.

In a further example not shown, different settings may be presented and selected from a list of vibrational event settings presented in the graphical user interface 410. For example, a duration of the vibrational event setting, a high, medium or low frequency range setting, and an identification of site-based setting adjustments to the vibrational event schedule may all be presented in the graphical user interface 410. Of course, the graphical user interface is not the only means to input user preferences or to establish the vibrational event schedule. A microphone or keyboard may be used to input user preferences. Alternatively, a computer-readable file containing the vibrational event schedule may be downloaded or transmitted wirelessly to the management device for storage in a database in the memory of the management device. Selections of settings in the site management application may be made upon receipt of an input selecting the particular setting.

Another example, the wearable drug delivery device may be configured to respond to a soft button on a graphical user interface, such as 410, presented on a management device, such as a smartphone or personal diabetes management device to remove the wearable drug delivery device with vibration, and after selecting the button, the wearable drug delivery device may be vibrate for a preset period of time, such as 5 seconds of the like, to allow the user time to remove the adhesive during the vibration event. As an alternative to the preset period of time, the wearable drug delivery device may, for example, be configured with a light sensor on the bottom of the wearable drug delivery device to indicate when the wearable drug delivery device has been removed from the body, and upon sensing the light, the vibration stops.

Returning to the example of FIG. 4A, based on user preference settings that may be set via the inputs received from the graphical user interface, the processor of the management device may be configured to establish a vibrational event schedule according to the user preferences and implement the established vibrational event schedule (435).

Of course, there are a myriad of other settings that may also be made. For example, another setting that may be presented for setting is a vibration direction, such as parallel vibrations, transverse vibrations or a combination of parallel vibrations and transverse vibrations. Each of the different vibration directions may provide a different benefit to the skin and/or underlying tissue at and/or around the attachment location of the wearable drug delivery device on the user.

In addition, identification of the attachment location may cause modifications to user preference selections as well as changes to default vibrational event schedule settings. In an example, each attachment location may have a vibrational event schedule specific to that attachment location. In an example, the processor of the management device may receive an orientation signal from the wearable drug delivery device. In response to the received orientation signal, the processor may be configured to determine an attachment location of the wearable drug delivery device on a user. Based on the determined attachment location, the processor may access a list of specific vibrational event schedules and select a vibrational event schedule corresponding to the determined attachment location.

Once the different settings for the respective vibrational event schedule are set, the site management application executing on the management device may begin monitoring the status of different components or elements of the wearable drug delivery device.

Since the wearable drug delivery device may have a limited power supply provided by its power source, it would be advantageous if the vibrational events schedules included specific settings that were automatically modified based on a power reserve of the power source of the wearable drug delivery device. For example, the duration and/or frequency of the vibrational events may be reduced if the power reserve is low or surpasses a threshold.

FIG. 4C illustrates an example process that modifies a vibrational event and a vibrational event schedule in response to a monitored condition of a wearable drug delivery device. The process 409 may be implemented by a site management application executing on a management device. In the example process 409, the controller of the wearable drug delivery device may be configured to monitor a power reserve of the power source by receiving signals from a device sensor coupled, for example, to a power supply of the wearable drug delivery device. The controller may be configured to forward an indication of the monitored power reserve to a management device for evaluation and analysis. The controller of the wearable drug delivery device may transmit, via a transceiver of the wearable drug delivery device, a signal containing an indication representative of a power reserve of the power source. Power reserve, in this example, may mean an amount of available energy or available electrical power. For example, a required power reserve for implementing different vibrational event schedules may be known for different times over a lifecycle of a wearable drug delivery device may be known and may be stored in a database of required power reserve in a memory of the management device. The database of required power reserve may be accessed and maintained by a site management application executing on the management device.

At 411, a transceiver of a management device may receive a signal and the indication of a power reserve of the power source of the wearable drug delivery device may be obtained from the signal by the processor of the management device. The indication may provide a value representative of an amount of the power reserve of the power source may be compared to a power reserve threshold value.

The processor may be configured to determine if the power source has enough of a power reserve to continue with a selected vibrational event schedule and also maintain the delivery of insulin according to a diabetes treatment plan set for the user (421). For example, the processor may be configured to access the database of required power reserve and look up the required power reserve that corresponds to the amount of time since the wearable drug delivery device was initiated for use by the user. The processor may initiate a clock or counter when the wearable drug delivery device was initiated for use. For example, time zero may correspond to the initiation time of the wearable drug delivery device when the power reserve may be some percentage (e.g., 50%) of the overall power of the power source. As time progresses and the wearable drug delivery device operates and delivers insulin and applies vibrational events, the electrical power or electrical energy in the power supply may have diminished since time zero. For example, after 1 day (i.e., time one), the percentage of overall power may be approximately 37% or the like. Using the known power reserve and power requirements for administering the basal dosages and bolus dosages, the processor may also adjust a power supply threshold at time progresses, such as from time zero to time one. For example, the processor may be configured to compare the indicated power reserve to a power supply threshold of the wearable drug delivery device. Alternatively, the processor may be configured to make a real time calculation of the required power reserve given the user's diabetes treatment plan, the amount of time left in the lifecycle of the pod, and the selected vibrational event schedule.

Based on the determination, at 421, that the power reserve is below the power reserve threshold value, the processor of the management device may output a signal causing a vibrational event schedule of vibrational events to be modified (431). Modifications to the vibrational event schedule may, for example, include extending the vibration intervals (i.e., the time between when vibrational events are administered) to make the intervals more intermittent when battery reserves are below a power reserve threshold. For example, the processor may receive an indication of a power reserve of a power source of the wearable drug delivery device. In the example, the indication provides a value representative of an amount of the power reserve of the power source. Based on the indication of the power reserve, the processor may select a modified duration of the vibrational event different from the duration of the vibrational event that was previously set, for example, in a vibrational event schedule. In addition, the processor may select, based on the indication of the power reserve, a modified vibration frequency of the vibrational event. In the example, the modified vibration frequency may include one or more vibration frequencies selected from a range of vibration frequencies different from the frequency of the vibrational event.

The site management application executing on the processor of the management device may be configured to make selections that modify a vibrational event that is next to be applied from the vibrational event schedule. For example, based on the indication of the power reserve, the site management cause the processor to modify a set duration of a vibrational event from the vibrational event schedule, a set vibration frequency of the vibrational event, and a set time when the vibrational event is to be applied. Any or all of the settings of a selected vibrational event schedule may be modified. The modified vibrational event schedule may be implemented at 441.

Further examples of controlling wearable devices, such as those in the examples of FIGs. 1B, 1D, 2A and 2B are also disclosed.

In an example of individual control of multiple vibrational actuators operating in cooperation with one another, the vibrational event may be considered to include the vibrations from all of the multiple vibrational actuators when they are operating in cooperation with one another. In an alternative example, a vibrational event may be defined for each respective vibrational actuator of the multiple vibrational actuators that are controlled to operate in cooperation.

In such an example of two vibrational actuators, the vibrational event may be considered to include both of the vibrational actuators. In the example, the management device processor when executing the site management code stored in a memory of the management device may be configured to select a first vibration frequency for the first vibrational actuator. Execution of the site management code may cause the processor to select a second vibration frequency for the second vibrational actuator. The selected first vibration frequency may be the same as the selected second vibration frequency. Alternatively, the selected first vibration frequency may be different from the selected second vibration frequency. In the example, when the selected first and selected second vibration frequencies are different, the vibrational actuators may be of different types of vibrational actuators (e.g., one being a piezo ceramic transducer and the other being an electric motor with eccentric weights). While the duration of the vibrational event may be set to a default value, the site management code may present on a graphical user interface an option for a user to select a duration of the vibrational event. In response to an input, the processor may select a duration for the vibrational event for the first vibrational actuator and the second vibrational actuator. In the example, selected duration for the vibrational event is the same for the first vibrational actuator and the second vibrational actuator. The site management code may also enable the processor to allow selection of a timing of the output of a control signal from the management device to the wearable drug delivery device.

In a further operational example, the site management code may obtain a determination of a level of physical activity of a user of the wearable drug delivery device. A determination of a level of physical activity may be obtained from health application, fitness application, pedometer application or the like of a management device. The site management code may determine a location of the wearable drug delivery device on the user, for example, based on signals from an ordinate sensor or input from the user. Based on the determination of the level of physical activity of the user and the location of the wearable drug delivery device on the user, the site management code may cause the processor to adjust a vibrational event schedule and vibrational event settings. For example, if the user is a physically active person and places the wearable drug delivery device on an attachment location that is their upper arm or thigh, movement from the physical activity (e.g., running, jumping, throwing, swinging a golf club or bowling ball) serves to stimulate tissue in areas around the wearable drug delivery device. As a result of the physical activity and the attachment location of the wearable drug delivery device, the site management code may cause the processor to adjust a vibrational event schedule, for example, by extending the vibration interval between application of vibrational events, and adjust vibrational event settings, such as vibration frequency and duration of the vibrational event. The attachment location may cause the processor of the management device to make site-based adjustments to the vibrational event schedule and any selected vibrational event settings.

Instead of relying on a user to select when a vibrational event is to take place, the site management code may automatically determine when a control signal is to be applied by a controller of the wearable drug delivery device to the vibrational actuators.

In an example of different types of vibrational actuators, the site management code may enable the processor to schedule a vibrational event that includes a determined timing of application of a control signal, the selected first vibration frequency, the selected second vibration frequency, and the selected duration in the command signal.

Alternatively, the processor of a management device may, based on sensor signals (e.g., a power reserve monitoring circuit signals, a blood glucose monitor signals, ordinate sensor signals, or the like) received from the wearable drug delivery device, determine a timing of application of a control signal to the first vibrational actuator and a timing of application of another control signal to the second vibrational actuator. The processor may output a command signal to the controller of the wearable drug delivery device for application of the vibrational event. The command signal may, for example, include first instructions for actuating the first vibrational actuator and second instructions for actuating a second vibrational actuator.

In an example, a personal diabetes management device, a smartphone, a smart accessory device (e.g., a smartwatch or other smart wearable device), or a combination of them may be configured to establish a wireless connection with a controller of a wearable drug delivery device. For example, the wearable drug delivery device may be equipped with a radio frequency transceiver or optical signal transceiver, configured to operate according to a wireless communication protocol, such as Bluetooth^{®}, Zigbee^{®}, Wi-Fi, a modulated optical signal, or the like. The respective personal diabetes management device, smartphone, or smart accessory device may, of course, be similarly equipped.

In an operational example, the management device processor, prior to outputting a command signal containing instructions for the controller of a wearable drug delivery device to actuate vibrational actuators to implement the vibrational event, may receive a notification that a bolus dosage is scheduled to be delivered by the wearable drug delivery device. In response to the notification, the processor using the information related to the settings (e.g., time interval, duration, frequencies and the like) corresponding to the vibrational event, determine when to output the command signal to the controller of the wearable drug delivery device.

For example, the processor may be configured by execution of the site management code to output the command signal within a preset time prior to delivery of the bolus dosage. The preset time prior to the delivery of the bolus dosage may, for example, be selected from a group of one or more preset times, such as 2 minutes, 1 minute, 30 seconds, 10 seconds, 2.5 seconds, 0.5 seconds, or the like.

It may be helpful to discuss an example of a drug delivery system that may implement the device and process examples of FIGs. 1A-4C.

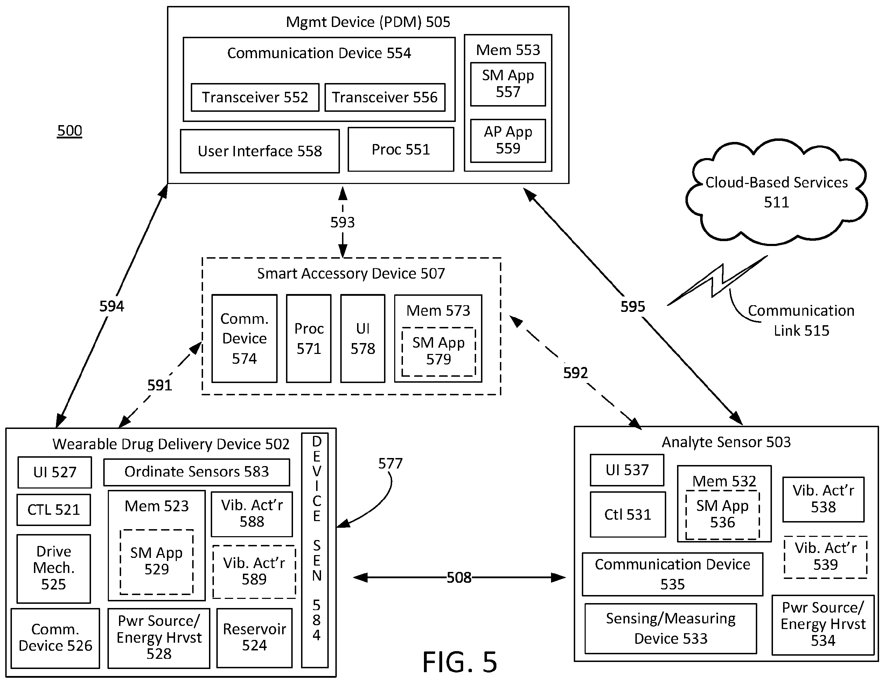

FIG. 5 illustrates a functional block diagram of a system example suitable for implementing the example processes and techniques described herein.

The drug delivery system 500 may implement an artificial pancreas (AP) algorithm (and/or provide AP functionality) to govern or control automated delivery of insulin to a user (e.g., to maintain euglycemia - a normal level of glucose in the blood). The drug delivery system 500 may be an automated drug delivery system that may include a wearable drug delivery device 502, an analyte sensor 503, and a management device (PDM) 505. The system 500, in an optional example, may also include a smart accessory device 507, such as a smartwatch, a personal assistant device or the like, which may communicate with the other components of system 500 via either a wired or wireless communication links 591-593.

In various examples described herein, the drug delivery system 500 may be configured to control the application of vibrations from the wearable drug delivery device 502 to an attachment location (shown in earlier examples) of the wearable drug delivery device. The application of the vibrations may be controlled by the management device 505 by implementing a vibrational event schedule in cooperation with a controller on the wearable drug delivery device 502.

The management device 505 may be a computing device such as a smart phone, a tablet, a personal diabetes management device, a dedicated diabetes therapy management device, or the like.

In an example, the management device (PDM) 505 may include a processor 551, a management device memory 553, a user interface 558, and a communication device 554. The management device 505 may contain analog and/or digital circuitry that may be implemented as a processor 551 for executing processes to manage a user's blood glucose levels and for controlling the delivery of the drug or therapeutic agent to the user as well as other functions, such as managing the implementation of vibrational events as discussed above. The management device 505 may be used to program or adjust operation of the wearable drug delivery device 502 and/or the analyte sensor 503.

The processor 551 may also be configured to execute programming code stored in the management device memory 553, such as an artificial pancreas application (AP app) 559 and site management application 557. The memory 553 may also store programming code to, for example, operate the user interface 558 (e.g., a touchscreen device, a camera or the like), the communication device 554 and the like. The processor 551 when executing the site management application 557 may be configured to implement a vibrational event schedule and provide other functions, such as indications and notifications related to vibrational events, and the like. The user interface 558 may under the control of the processor 551 be configured to present a graphical user interface that enables the input of vibrational event setting selections and the like as described above.

The processor 551 is also configured to execute a diabetes treatment plan that is managed by the artificial pancreas (AP) application 559 stored in memory 553. The AP application 559 provides functionality to enable the processor 551 to determine a bolus dosage and determine a basal dosage according to a diabetes treatment plan. In addition, the AP application 559 provides functionality to enable the processor 551 to output signals to the wearable drug delivery device 502 to deliver the determined bolus and basal dosages.

The communication device 554 may include one or more transceivers such as 552 and 556 and receivers or transmitters that operate according to one or more radio-frequency protocols. In the example, the transceivers 552 and 556 may be a cellular transceiver and a Bluetooth^{®} transceiver, respectively. For example, the communication device 554 may include a transceiver 552 or 556 configured to receive and transmit signals containing information usable by the artificial pancreas application 559 and the site management application 557.

In the example, the wearable drug delivery device 502 may include a user interface 527, a controller 521, a drive mechanism 525, a communication device 526, an ordinate sensor(s) 583, a memory 523, a power source/energy harvesting circuit 528 (or more, generally, power source), actuator 588, an optional actuator 589 (depending upon implementation as shown in earlier examples), device sensors 584, and a reservoir 524.

The memory 523 may store programming code executable by the controller 521. The programming code, for example, may enable the controller 521 to control expelling insulin from the reservoir 524 and control the administering of vibrations by controlling actuation of the vibrational actuator 588 and, if so equipped, optional vibrational actuator 589. The memory 523 may also store an instance of the site management (SM) application 529. Execution of the SM application 529 may enable the controller 521 to respond to command signals from the management device 505. The memory 523 may store a vibrational event schedule, which may be a vibrational event schedule is a list of the vibrational events with times, durations and frequency of the vibrations for each vibrational event and may list all vibrational events for a number of hours, a day, several days, a lifecycle of a wearable drug delivery device, or the like.

The reservoir 524 may be configured to store drugs or therapeutic agents, such as insulin, morphine or the like, suitable for automated delivery. The device sensors 584 may include one or more of a pressure sensor, a power source/energy harvesting circuit sensor, or the like that are communicatively coupled to the controller 521 and provide various signals. For example, a pressure sensor of the device sensors 584 may be configured to provide an indication of the fluid pressure detected in a fluid pathway between a needle or cannula (shown in examples of FIGs. 2A and 2B)) inserted in a user and the reservoir 524. For example, the pressure sensor may be coupled to or integral with a needle/cannula insertion component or the like. In an example, the controller 521 or a processor, such as 551, may be operable to determine that a rate of drug infusion based on the indication of the fluid pressure. The rate of drug infusion may be compared to an infusion rate threshold. In response to a determination that the rate of drug infusion is less than an infusion rate threshold, a control signal may be output to actuate the vibrational actuator.

The power source/energy harvesting circuit sensor may output an indication of an amount of power left (e.g., a power reserve) of the power source or energy harvesting circuit (not shown in these examples).

In an example, the wearable drug delivery device 502 includes a communication device 526, which may be a receiver, a transmitter, or a transceiver that operates according to one or more radio-frequency protocols, such as Bluetooth, Wi-Fi, a near-field communication standard, a cellular standard, or the like.

The controller 521 may, for example, communicate with a personal diabetes management device 505 and an analyte sensor 503 via a transceiver (not shown in this example).

In an example, the controller 521 of the wearable drug delivery device 502 when executing the site management application 529 may make decisions when the management device 505 is not within range to establish via a wireless communication link, such as 594. For example, the controller may monitor the power reserve of the power source and may not be configured to send an indication of the monitored power reserve to the management device 505. In some examples, the controller 521 may override a command signal received from the management device 505 when the power reserve is below the power reserve threshold. In another example, the number of times or duration of the vibrational events may be stored in the memory 523 and shared with the management device 505. The site management application 557 may in combination with user feedback utilize an adaptive model to modify a vibrational event schedule to increase or decrease the number or duration of the vibrational events in the vibrational event schedule selected for application to the user.

The site management application 529 may cause the controller 521 to generate vibrations in response to a user input, a schedule as described with reference to other examples or the like. A user input may be, for example, received via the user interface 527. For example, a user may wish to remove the wearable drug delivery device 502 from an attachment location and via the user interface 527, signal the controller 521 of the intention to remove the wearable drug delivery device 502. In response to the received signal, the site management application 529 may cause the controller 521 to actuate the vibrational actuators 588 and 589 to assist with the removal of the wearable drug delivery device 502. Alternatively, the site management application 529 may cause the controller 521 to actuate the vibrational actuators 588 and 589 to assist with maintaining the accuracy of the detection of the analyte characteristics of the user. In addition, or alternatively, the user may utilize a graphical user interface on a management device 505 such as graphical user interface 410 shown in the example of FIG. 4B. In place of inputs 412, 414 and 416, the user input may be an indication that the user intends to remove the wearable drug delivery device 502 from the skin surface (e.g., "Remove Sensor" or the like). The management device 505 may send a signal to the wearable drug delivery device 502, which receives the signal via communication device 526. The controller 521 coupled to the communication device 526 may process the signal and the site management application 529 may actuate a respective vibrational actuators 588 or 589 or both to provide vibrations (for a set period of time, such as, for example, 5 or 10 seconds) to alleviate pain during removal or aid removal of the wearable drug delivery device 502.

The wearable drug delivery device 502 may be attached to the body of a user, such as a patient or diabetic, at an attachment location and may deliver any therapeutic agent, including any drug or medicine, such as insulin or the like, to a user at or around the attachment location. A surface of the wearable drug delivery device 502 may include an adhesive to facilitate attachment to the skin of a user as described in earlier examples.

In an example, the ordinate sensors 583 positioned within a housing 577 of the wearable drug delivery device 502 may be operable to output an ordinate sensor signal or signals that the controller 521, which may use to determine an attachment location on a user's body. Alternatively, the controller 521 may forward the ordinate sensor signals to the management device 505, where processor 551 may be operable to use the received ordinate sensor signals to determine an attachment location on a user's body. In another example, the communication between the controller 521, the personal diabetes management device 505, the analyte sensor 503 may enable the user to manually input the attachment location (e.g., thigh, upper arm, abdomen, or the like) of wearable drug delivery device 502 on the user's body via a graphical user interface. In response to the user manually entering the attachment location of the wearable drug delivery device 502, the controller 521 may be operable to use the ordinate sensor signals received from the ordinate sensors 583 to confirm that the manually entered attachment location of the wearable drug delivery device 505 corresponds to the ordinate sensor signals typically output by the ordinate sensors 583.

The wearable drug delivery device 502 may, for example, include a reservoir 524 for storing the drug (such as insulin), a needle or cannula (not shown in this example) for delivering the drug into the body of the user (which may be done subcutaneously, intraperitoneally, or intravenously), and a drive mechanism 525 for transferring the drug from the reservoir 524 through a needle or cannula and into the user. The drive mechanism 525 may be fluidly coupled to reservoir 524, and communicatively coupled to the controller 521.

The wearable drug delivery device 502 may also include a power source 528, such as a battery, a piezoelectric device, other forms of energy harvesting devices, or the like, for supplying electrical power to the drive mechanism 525 and/or other components (such as the controller 521, memory 523, and the communication device 526) of the wearable drug delivery device 502.

In some examples, the wearable drug delivery device 502 and/or the management device 505 may include a user interface 558, respectively, such as a keypad, a touchscreen display, levers, light-emitting diodes, buttons on a housing of the management device 505, a microphone, a camera, a speaker, a display, or the like, that is configured to allow a user to enter information and allow the management device 505 to output information for presentation to the user (e.g., alarm signals or the like). The user interface 558 may provide inputs, such as a voice input, a gesture (e.g., hand or facial) input to a camera, swipes to a touchscreen, or the like, to processor 551 which the programming code interprets.

Instructions, such as command signals or actuation signals, may be transmitted to the wearable drug delivery device 502 over the wired or wireless link 594 by the management device (PDM) 505. The wearable drug delivery device 502 may execute any received instructions for administering the vibrational events and the delivery of a drug to the user according to a diabetes treatment plan.

In an operational example, the processor 551 when executing the site management application 557 may output a command signal via the communication device 554. The command signal may be based on the vibrational event schedule stored in memory 553. The command signal may include instructions for a controller (CTL) 521 of a wearable drug delivery device 502 to actuate vibrational actuators, such as 588 and/or 589 as described above.

The smart accessory device 507 may be, for example, an Apple Watch^{®}, other wearable smart device, including eyeglasses, provided by other manufacturers, a global positioning system-enabled wearable, a wearable fitness device, smart clothing, or the like. Similar to the management device 505, the smart accessory device 507 may also be configured to perform various functions including controlling the wearable drug delivery device 502. For example, the smart accessory device 507 may include a communication device 574, a processor 571, a user interface 578 and a memory 573. The user interface 578 may be a graphical user interface presented on a touchscreen display of the smart accessory device 507. The memory 573 may store programming code to operate different functions of the smart accessory device 507 as well as an instance of the site management application 579. The processor 571 that may execute programming code, such as site management (SM) App 579 for controlling the wearable drug delivery device 502 to implement the described examples of a vibrational event and vibrational event scheduling processes described herein.

The analyte sensor 503 may include a controller 531, a memory 532, a sensing/measuring device 533, a user interface 537, a vibrational actuator 538, an optional vibrational actuator 539, a power source/energy harvesting circuitry 534, and a communication device 535. The analyte sensor 503 may be communicatively coupled to the processor 551 of the management device 505 or controller 521 of the wearable drug delivery device 502. The memory 532 may be configured to store information and programming code, such as site management application 536.

The analyte sensor 503 may be configured to detect multiple different analytes, such as lactate, ketones, sodium, potassium, uric acid, alcohol levels or the like, and output results of the detections, such as measurement values or the like. The analyte sensor 503 may, in an example, be configured to measure a blood glucose value at a predetermined time interval, such as every 5 minutes, or the like. The communication device 535 of analyte sensor 503 may have circuitry that operates as a transceiver for communicating the measured blood glucose values to the management device 505 over a wireless link 595 or with wearable drug delivery device 502 over the wireless communication link 508. While called an analyte sensor 503 includes a glucose measurement element, the sensing/measuring device 533 of the analyte sensor 503 may include one or more additional sensing elements, such as a heart rate monitor, a pressure sensor, or the like. The controller 531 may include discrete, specialized logic and/or components, an application-specific integrated circuit, a microcontroller or processor that executes software instructions, firmware, programming instructions stored in memory (such as memory 532), or any combination thereof.

Similar to the controller 521, the controller 531 may be operable to perform many functions. For example, the controller 531 may be configured by the programming code stored in the memory 532 to manage the collection and analysis of data detected the sensing and measuring device 533 as well as execute the site management application 536 to control operation of the vibrational actuators 538 and 539. The site management application 536 may cause the controller 531 to generate vibrations in response to a user input, a schedule as described with reference to other examples or the like. A user input may be, for example, received via the user interface 537. For example, a user may wish to remove the analyte sensor 503 from an attachment location and via the user interface 537, signal the controller 531 of the intention to remove the analyte sensor 503. In response to the received signal, the site management application 536 may cause the controller 531 to actuate the vibrational actuators 538 and 539 to assist with the removal of the analyte sensor 503. Alternatively, the site management application 536 may cause the controller 531 to actuate the vibrational actuators 538 and 539 to assist with maintaining the accuracy of the detection of the analyte characteristics, analyte attributes, or analyte levels of the user. In addition, or alternatively, the user may utilize a graphical user interface on a management device 505 such as graphical user interface 410 shown in the example of FIG. 4B. In place of inputs 412, 414 and 416, the user input may be an indication that the user intends to remove the analyte sensor 503 from the skin surface (e.g., "Remove Sensor" or the like). The management device 505 may send a signal to the analyte sensor 503, which receives the signal via communication device 535. The controller 531 coupled to the communication device 535 may process the signal and the site management application 536 may actuate a respective vibrational actuators 538 or 539 or both to provide vibrations (for a set period of time, such as, for example, 5 or 10 seconds) to aid removal of the analyte sensor 503.

Although the analyte sensor 503 is depicted as separate from the wearable drug delivery device 502, in various examples, the analyte sensor 503 and wearable drug delivery device 502 may be incorporated into the same unit. That is, in various examples, the sensor 503 may be a part of the wearable drug delivery device 502 and contained within the same housing of the wearable drug delivery device 502 (e.g., the sensor 503 may be positioned within or integrated into the wearable drug delivery device 502).

The communication link 515 that couples the cloud-based services 511 to the respective devices 502, 503, 505 or 507 of system 500 may be a cellular link, a Wi-Fi link, a Bluetooth link, or a combination thereof. Services provided by cloud-based services 511 may include data storage that stores anonymized data, such as blood glucose measurement values, vibrational event schedules, and, perhaps, other forms of data. In addition, the cloud-based services 511 may process the anonymized data from multiple users to provide generalized information related to the various settings used by the site management application, such as default settings for a vibrational event schedule.

The wireless communication links 508, 591, 592, 593, 594 and 595 may be any type of wireless link operating using known wireless communication standards or proprietary standards. As an example, the wireless communication links 508, 591, 592, 593, 594 and 595 may provide communication links based on Bluetooth^{®}, Zigbee^{®}, Wi-Fi, a near-field communication standard, a cellular standard, or any other wireless protocol via the respective communication devices 554, 574, 526 and 535.

Some examples of the disclosed device may be implemented, for example, using a storage medium, a computer-readable medium, or an article of manufacture which may store an instruction or a set of instructions that, if executed by a machine (i.e., processor or microcontroller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

Program aspects of the technology may be thought of as "products" or "articles of manufacture" typically in the form of executable code and/or associated data that is carried on or embodied in a type of machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but by the claims appended hereto. Future filed applications claiming priority to this application may claim the disclosed subject matter in a different manner and may generally include any set of one or more limitations as variously disclosed or otherwise demonstrated herein.
Although the present invention is defined in the attached claims, it should be understood that the present invention can also (alternatively) be defined in accordance with the following embodiments:
1. A wearable drug delivery device, comprising:
   a controller configured to output control signals;
   a memory coupled to the controller and configured to store programming code, and a site maintenance application, wherein the programming code and the site maintenance application are executable by the controller;
   a vibrational actuator coupled to the controller and configured to generate vibrations in response to a control signal from the controller;
   a reservoir configured to store a therapeutic drug; and
   at least one housing configured to contain the controller, the memory, the vibrational actuator, and the reservoir, and wherein an adhesive layer disposed on a bottom surface of the at least one housing which is configured to affix to skin of a user, and wherein the controller when executing the site maintenance application is configured to:
      control the vibrational actuator to generate vibrations that extend below the bottom surface, wherein the generated vibrations have a duration and a frequency.
2. The wearable drug delivery device of embodiment 1, wherein the vibrational actuator is positioned at an angle with respect to the bottom surface.
3. The wearable drug delivery device of embodiment 1, wherein the vibrational actuator is positioned within the at least one housing and operable to transmit the generated vibrations either transverse or parallel to a central axis of the at least one housing.
4. The wearable drug delivery device of embodiment 1, wherein the vibrational actuator is a piezo ceramic transducer controllable to transmit the generated vibrations at a frequency between approximately 1 MHz and approximately 10 MHz.
5. The wearable drug delivery device of embodiment 1, wherein the vibrational actuator is an electric motor having a shaft and an eccentric weight coupled to the shaft of the electric motor, and controllable to transmit the generated vibrations at a frequency up to approximately 300 Hz.
6. The wearable drug delivery device of embodiment 1, wherein the vibrational actuator is a first vibrational actuator and a second vibrational actuator.
7. The wearable drug delivery device of embodiment 6, wherein:
   the first vibrational actuator is positioned within the at least one housing to transmit, when actuated, vibrations in a first direction that are substantially parallel to a central axis of the at least one housing,
   the second vibrational actuator is positioned within the at least one housing to transmit vibrations, when actuated, in a second direction that are substantially parallel to the central axis of the at least one housing, and
   the first direction is opposite to the second direction and the first vibrational actuator and second vibrational are alternately actuated.
8. The wearable drug delivery device of embodiment 6, wherein:
   the first vibrational actuator and the second vibrational actuator are controllable to be simultaneously actuated to transmit the generated vibrations in a direction that is transverse to a central axis of the at least one housing; or
   the first vibrational actuator and the second vibrational actuator are positioned at opposing angles that enable the controller to control the first vibrational actuator and the second vibrational actuator to generate variable vibration patterns.
9. The wearable drug delivery device of embodiment 6, wherein the first vibrational actuator is a piezo ceramic transducer, and the second vibrational actuator is an electric motor having a shaft and an eccentric weight coupled to the shaft of the electric motor.
10. The wearable drug delivery device of embodiment 1, wherein the controller is further operable to output a modulated control signal to the vibrational actuator, and in response to the modulated control signal, the vibrational actuator is configured to generate modulated vibrations.
11. The wearable drug delivery device of embodiment 1, wherein the controller is further operable to:
   determine that a rate of drug infusion is less than an infusion rate threshold; and
   based on the determination, output the control signal to actuate the vibrational actuator.
12. The wearable drug delivery device of embodiment 1, further comprising:
   two or more ordinate sensors located at fixed positions within the at least one housing, wherein each respective ordinate sensor of the two or more ordinate sensors is operable to output a signal indicating a respective orientation; and
   wherein the controller is operable to:
      receive the signal output from each respective ordinate sensor of the two or more ordinate sensors;
      determine an attachment location of the wearable drug delivery device on the user; and
      output an indication of the attachment location to cause selection of a vibrational event schedule.
13. The wearable drug delivery device of embodiment 12, wherein the controller is operable to:
   receive an ordinate sensor signal output from an ordinate sensor positioned in the at least one housing; and
   output the ordinate sensor signal to be transmitted via a transceiver to a management device that is external to the wearable drug delivery device.
14. A non-transitory computer readable medium embodied with programming code executable by a processor, and the processor when executing the programming code is operable to:
   establish a wireless connection with a controller of a wearable drug delivery device;
   determine settings to implement a vibrational event for the wearable drug delivery device, wherein the determined settings include a duration of the vibrational event and a frequency of a vibration to be generated during the vibrational event; and
   output a command signal containing instructions for the controller of a wearable drug delivery device to actuate vibrational actuators to implement the vibrational event according to the determined settings within the wearable drug delivery device.
15. The non-transitory computer readable medium of embodiment 14, further embodied with programming code executable by the processor, and the processor, when executing the programming code to determine the settings to implement the vibrational event, is operable to:
   access a database storing information related to the settings associated with the vibrational event to be implemented on the wearable drug delivery device, wherein the information related to the settings includes user preferences related to the vibrational event, wearable drug delivery device sensor indications, or time of day; and
   using the information related to the settings, determine when to output the command signal to the controller of the wearable drug delivery device.
16. The non-transitory computer readable medium of embodiment 14, further embodied with programming code executable by the processor, and the processor when executing the programming code is operable to:
   receive an orientation signal from the wearable drug delivery device;
   determine, in response to the received orientation signal, an attachment location of the wearable drug delivery device on a user; and
   select a vibrational event schedule corresponding to the determined attachment location.
17. The non-transitory computer readable medium of embodiment 14, further embodied with programming code executable by the processor, and the processor when executing the programming code is operable to:
   output a command signal to the controller of the wearable drug delivery device, wherein the command signal includes first instructions for actuating a first vibrational actuator and second instructions for actuating a second vibrational actuator.
18. The non-transitory computer readable medium of embodiment 14, further embodied with programming code executable by the processor, and the processor, when executing the programming code, is operable to:
   establish a vibrational event schedule according to user preferences, wherein the user preferences enable scheduling of the vibrational event at specific times during a day and selection of vibrational event settings, wherein:
   establishing the schedule of the vibrational event includes selection of daylight hours during which the vibrational event is administered, selection of evening hours during which the vibrational event is administered, or a combination of both daylight hours and evening hours; and
   selection of vibrational event settings includes selection of a duration of the vibrational event, selection of a high frequency range, a medium frequency range, or a low frequency range, and identification of site-based adjustments to the vibrational event schedule and the selected vibrational event settings,
   wherein the outputting of the command signal is based on the vibrational event schedule for the vibrational event to be implemented.
19. The non-transitory computer readable medium of embodiment 14, further embodied with programming code executable by the processor, and the processor, when executing the programming code, is operable to:
   obtain a determination of a level of physical activity of a user of the wearable drug delivery device;
   determine a location of the wearable drug delivery device on the user; and
   adjust a vibrational event schedule and vibrational event settings based on the determination of the level of physical activity of the user and the determined location of the wearable drug delivery device on the user.
20. A system, comprising:
   a personal diabetes management device, the personal diabetes management device including:
      a processor;
      a memory storing programming code and a site maintenance application, wherein the programming code and the site maintenance application are executable by the processor;
      a touchscreen display device coupled to the processor and configured to receive inputs and present a graphical user interface; and
      a transceiver coupled to the processor and be configured to receive and
   transmit signals containing information of the site maintenance application; and a wearable drug delivery device, the wearable drug delivery device including:
      a drug delivery device transceiver configured to be coupled via a wireless communication link with the personal diabetes management device;
      a reservoir configured to contain insulin;
      a drive mechanism coupled to the reservoir and configured to expel the insulin from the reservoir;
      a vibrational actuator configured to generate vibrations; and
      a controller coupled to the drug delivery device transceiver, the drive mechanism and the vibrational actuator, wherein the controller is configured to
   receive command signals from the personal diabetes management device, and wherein the processor of the personal diabetes management device when executing programming code is configured to:
      prior to transmitting a command signal causing the drive mechanism to deliver a bolus dosage of insulin from the reservoir, transmit, according to a vibrational event schedule, a vibrational event command signal to the wearable drug delivery device, wherein the vibrational event command signal includes a vibration duration and a vibration frequency; and
   wherein the controller of the wearable drug delivery device is configured to:
   in response to the vibrational event command signal, send a control signal to actuate the vibrational actuator.

## Claims

1. A wearable drug delivery device, comprising:
a controller configured to output control signals;
a memory coupled to the controller and configured to store programming code, and a site maintenance application, wherein the programming code and the site maintenance application are executable by the controller;
a vibrational actuator coupled to the controller and configured to generate vibrations in response to a control signal from the controller;
a reservoir configured to store a therapeutic drug; and
at least one housing configured to contain the controller, the memory, the vibrational actuator, and the reservoir, and wherein an adhesive layer disposed on a bottom surface of the at least one housing which is configured to affix to skin of a user, and
wherein the controller when executing the site maintenance application is configured to:
control the vibrational actuator to generate vibrations that extend below the bottom surface, wherein the generated vibrations have a duration and a frequency.

2. The wearable drug delivery device of claim 1, wherein the vibrational actuator is positioned at an angle with respect to the bottom surface.

3. The wearable drug delivery device of claim 1, wherein the vibrational actuator is positioned within the at least one housing and operable to transmit the generated vibrations either transverse or parallel to a central axis of the at least one housing.

4. The wearable drug delivery device of claim 1, wherein the vibrational actuator is a piezo ceramic transducer controllable to transmit the generated vibrations at a frequency between approximately 1 MHz and approximately 10 MHz.

5. The wearable drug delivery device of claim 1, wherein the vibrational actuator is an electric motor having a shaft and an eccentric weight coupled to the shaft of the electric motor, and controllable to transmit the generated vibrations at a frequency up to approximately 300 Hz.

6. The wearable drug delivery device of claim 1, wherein the vibrational actuator is a first vibrational actuator and a second vibrational actuator.

7. The wearable drug delivery device of claim 6, wherein:
the first vibrational actuator is positioned within the at least one housing to transmit, when actuated, vibrations in a first direction that are substantially parallel to a central axis of the at least one housing,
the second vibrational actuator is positioned within the at least one housing to transmit vibrations, when actuated, in a second direction that are substantially parallel to the central axis of the at least one housing, and
the first direction is opposite to the second direction and the first vibrational actuator and second vibrational are alternately actuated.

8. The wearable drug delivery device of claim 6, wherein:
the first vibrational actuator and the second vibrational actuator are controllable to be simultaneously actuated to transmit the generated vibrations in a direction that is transverse to a central axis of the at least one housing; or
the first vibrational actuator and the second vibrational actuator are positioned at opposing angles that enable the controller to control the first vibrational actuator and the second vibrational actuator to generate variable vibration patterns.

9. The wearable drug delivery device of claim 6, wherein the first vibrational actuator is a piezo ceramic transducer, and the second vibrational actuator is an electric motor having a shaft and an eccentric weight coupled to the shaft of the electric motor.

10. The wearable drug delivery device of claim 1, wherein the controller is further operable to output a modulated control signal to the vibrational actuator, and in response to the modulated control signal, the vibrational actuator is configured to generate modulated vibrations.

11. The wearable drug delivery device of claim 1, wherein the controller is further operable to: determine that a rate of drug infusion is less than an infusion rate threshold; and based on the determination, output the control signal to actuate the vibrational actuator.

12. The wearable drug delivery device of one of claims 1 to 11, further comprising a pressure sensor for providing an indication of the fluid pressure detected in a fluid pathway between a needle or cannula to be inserted in a user and the reservoir.

13. The wearable drug delivery device of claim 12, wherein the controller or a processor is operable to determine a rate of drug infusion based on the indication of the fluid pressure.

14. The wearable drug delivery device of one of claims 1 to 11, further comprising a pressure sensor, wherein, if a detected pressure within a fluid pathway exceeds a threshold, then a vibrational event is automatically triggered in an effort to reduce the amount of pressure within the fluid pathway.

15. The wearable drug delivery device of claim 1, further comprising:
two or more ordinate sensors located at fixed positions within the at least one housing, wherein each respective ordinate sensor of the two or more ordinate sensors is operable to output a signal indicating a respective orientation; and
wherein the controller is operable to:
receive the signal output from each respective ordinate sensor of the two or more ordinate sensors;
determine an attachment location of the wearable drug delivery device on the user; and
output an indication of the attachment location to cause selection of a vibrational event schedule.
